# EUROPEAN PATENT APPLICATION

(11) **EP 3 872 492 A1**
(43) Date of publication of application: **01.09.2021**
(21) Application number: 20173061.1
(22) Date of filing: 05.05.2020
(51) Int. Cl.: G01N 33/569, C07K 14/005

(54) **IDENTIFYING BORNA DISEASE VIRUS (BODV) INFECTION THROUGH DETECTION OF ANTIBODIES AGAINST THE BODV GLYCOPROTEIN**

(30) Priority: 28.02.2020 EP 20160121
(71) Applicant: Seramun Diagnostica GmbH, 15754 Heidesee (DE)
(72) Inventor: Schlegel, Mathias, 15344 Strausberg (DE); Faupel, Thomas, 10777 Berlin (DE); Löster, Klemens, 16562 Hohen Neuendorf (DE)
(74) Representative: Hertin und Partner Rechts- und Patentanwälte PartG mbB

(57) **Abstract**

The invention relates to a solid phase comprising one or more immobilized Borna disease virus (BoDV) antigens for the detection of BoDV specific antibodies in a sample, wherein said one or more immobilized BoDV antigens comprise BoDV-glycoprotein (BoDV-GP) and/or fragments thereof. In embodiments, the solid phase additionally comprises at least one antigen selected from the group comprising BoDV-N-protein, BoDV-P-protein and BoDV-X-protein, and/or fragments thereof, wherein preferably each antigen is located on a spatially separated area of the solid surface enabling individual and/or independent detection of antibodies directed against each antigen. The invention further relates to a method and kit for the detection of BoDV specific antibodies and/or BoDV infection.

SEQUENCE LISTING

<110> Seramun Diagnostica GmbH

<120> IDENTIFYING BORNA DISEASE VIRUS (BoDV) INFECTION THROUGH DETECTION OF
ANTIBODIES AGAINST THE BoDV GLYCOPROTEIN

<130> 2011/20EP

<150> EP20160121
<151> 2020-02-28

<160> 20

<170> BiSSAP 1.3.6

<210> 1
<211> 28
<212> DNA
<213> Artificial Sequence

<220>
<223> BoDV_P_aa001_Fwd

<400> 1
gagtggatcc atggcaacgc gaccatcg                                          28

<210> 2
<211> 37
<212> DNA
<213> Artificial Sequence

<220>
<223> BoDV_P_aa201_Rev

<400> 2
catagagtcg acttatggta tgatgtccca ttcatcc                                37

<210> 3
<211> 28
<212> DNA
<213> Artificial Sequence

<220>
<223> BoDV_N_aa001_Fwd

<400> 3
gagtggatcc atgccaccca agagacgc                                          28

<210> 4
<211> 38
<212> DNA
<213> Artificial Sequence

<220>
<223> BoDV_N_aa370_Rev

<400> 4
catagagtcg acttagttta gaccagtcac acctatca                                38

<210> 5
<211> 28
<212> DNA
<213> Artificial Sequence

<220>
<223> BoDV_gp_aa023_Fwd

<400> 5
gcgtctagac ttcgatctgc agggcctg                                           28

<210> 6
<211> 29
<212> DNA
<213> Artificial Sequence

<220>
<223> BoDV_gp_aa446_Rev

<400> 6
ctaggtaccg ctaccagtgt ctgtgctcg                                          29

<210> 7
<211> 460
<212> PRT
<213> Artificial Sequence

<220>
<223> BoDV-1.gp_aa23-446 construct expressed from
      pLEXSY-sat2.1_BoDV-1.gp_aa23-446

<400> 7
Met Ala Ser Arg Leu Val Arg Val Leu Ala Ala Ala Met Leu Val Ala
1               5                   10                  15
Ala Ala Val Ser Val Asp Ala Gly Ala Ser Leu Asp Phe Asp Leu Gln
            20                  25                  30
Gly Leu Ser Cys Asn Thr Asp Ser Thr Pro Gly Leu Ile Asp Leu Glu
        35                  40                  45
Ile Arg Arg Leu Cys His Thr Pro Thr Glu Asn Val Ile Ser Cys Glu
    50                  55                  60
Val Ser Tyr Leu Asn His Thr Thr Ile Ser Leu Pro Ala Val His Thr
65                  70                  75                  80
Ser Cys Leu Lys Tyr His Cys Lys Thr Tyr Trp Gly Phe Phe Gly Ser
                85                  90                  95
Tyr Ser Ala Asp Arg Ile Ile Asn Arg Tyr Thr Gly Thr Val Lys Gly
            100                 105                 110
Cys Leu Asn Asn Ser Ala Pro Glu Asp Pro Phe Glu Cys Asn Trp Phe
        115                 120                 125
Tyr Cys Cys Ser Ala Ile Thr Thr Glu Ile Cys Arg Cys Ser Ile Thr
    130                 135                 140
Asn Val Thr Val Ala Val Gln Thr Phe Pro Pro Phe Met Tyr Cys Ser
145                 150                 155                 160
Phe Ala Asp Cys Ser Thr Val Ser Gln Gln Glu Leu Glu Ser Gly Lys

                165                 170                 175
Ala Met Leu Ser Asp Gly Ser Thr Leu Thr Tyr Thr Pro Tyr Ile Leu
            180                 185                 190
Gln Ser Glu Val Val Asn Lys Thr Leu Asn Gly Thr Ile Leu Cys Asn
        195                 200                 205
Ser Ser Ser Lys Ile Val Ser Phe Asp Glu Phe Arg Arg Ser Tyr Ser
    210                 215                 220
Leu Thr Asn Gly Ser Tyr Gln Ser Ser Ser Ile Asn Val Thr Cys Ala
225                 230                 235                 240
Asn Tyr Thr Ser Ser Cys Arg Pro Arg Leu Lys Arg Arg Arg Arg Asp
                245                 250                 255
Thr Gln Gln Ile Glu Tyr Leu Val His Lys Leu Arg Pro Thr Leu Lys
            260                 265                 270
Asp Ala Trp Glu Asp Cys Glu Ile Leu Gln Ser Leu Leu Leu Gly Val
        275                 280                 285
Phe Gly Thr Gly Ile Ala Ser Ala Ser Gln Phe Leu Arg Ser Trp Leu
    290                 295                 300
Asn His Pro Asp Ile Ile Gly Tyr Ile Val Asn Gly Val Gly Val Val
305                 310                 315                 320
Trp Gln Cys His Arg Val Asn Val Thr Phe Met Ala Trp Asn Glu Ser
                325                 330                 335
Thr Tyr Tyr Pro Pro Val Asp Tyr Asn Gly Arg Lys Tyr Phe Leu Asn
            340                 345                 350
Asp Glu Gly Arg Leu Gln Thr Asn Thr Pro Glu Ala Arg Pro Gly Leu
        355                 360                 365
Lys Arg Val Met Trp Phe Gly Arg Tyr Phe Leu Gly Thr Val Gly Ser
    370                 375                 380
Gly Val Lys Pro Arg Arg Ile Arg Tyr Asn Lys Thr Ser His Asp Tyr
385                 390                 395                 400
His Leu Glu Glu Phe Glu Ala Ser Leu Asn Met Thr Pro Gln Thr Ser
                405                 410                 415
Ile Ala Ser Gly His Glu Thr Asp Pro Ile Asn His Ala Tyr Gly Thr
            420                 425                 430
Gln Ala Asp Leu Leu Pro Tyr Thr Arg Ser Ser Asn Ile Thr Ser Thr
        435                 440                 445
Asp Thr Gly Ser Gly Thr His His His His His His
    450                 455                 460

<210> 8
<211> 503
<212> PRT
<213> Borna disease virus

<220>
<223> BoDV-1-GP p57; aa 1-503

<400> 8
Met Gln Pro Ser Met Ser Phe Leu Ile Gly Phe Gly Thr Leu Val Leu
1               5                   10                  15
Val Leu Ser Ala Arg Thr Phe Asp Leu Gln Gly Leu Ser Cys Asn Thr
            20                  25                  30
Asp Ser Thr Pro Gly Leu Ile Asp Leu Glu Ile Arg Arg Leu Cys His
        35                  40                  45
Thr Pro Thr Glu Asn Val Ile Ser Cys Glu Val Ser Tyr Leu Asn His
    50                  55                  60
Thr Thr Ile Ser Leu Pro Ala Val His Thr Ser Cys Leu Lys Tyr His
65                  70                  75                  80
Cys Lys Thr Tyr Trp Gly Phe Phe Gly Ser Tyr Ser Ala Asp Arg Ile
                85                  90                  95
Ile Asn Arg Tyr Thr Gly Thr Val Lys Gly Cys Leu Asn Asn Ser Ala
            100                 105                 110
Pro Glu Asp Pro Phe Glu Cys Asn Trp Phe Tyr Cys Cys Ser Ala Ile

115                 120                 125
Thr Thr Glu Ile Cys Arg Cys Ser Ile Thr Asn Val Thr Val Ala Val
    130                 135                 140
Gln Thr Phe Pro Pro Phe Met Tyr Cys Ser Phe Ala Asp Cys Ser Thr
145                 150                 155                 160
Val Ser Gln Gln Glu Leu Glu Ser Gly Lys Ala Met Leu Ser Asp Gly
                165                 170                 175
Ser Thr Leu Thr Tyr Thr Pro Tyr Ile Leu Gln Ser Glu Val Val Asn
            180                 185                 190
Lys Thr Leu Asn Gly Thr Ile Leu Cys Asn Ser Ser Ser Lys Ile Val
        195                 200                 205
Ser Phe Asp Glu Phe Arg Arg Ser Tyr Ser Leu Thr Asn Gly Ser Tyr
    210                 215                 220
Gln Ser Ser Ser Ile Asn Val Thr Cys Ala Asn Tyr Thr Ser Ser Cys
225                 230                 235                 240
Arg Pro Arg Leu Lys Arg Arg Arg Arg Asp Thr Gln Gln Ile Glu Tyr
                245                 250                 255
Leu Val His Lys Leu Arg Pro Thr Leu Lys Asp Ala Trp Glu Asp Cys
            260                 265                 270
Glu Ile Leu Gln Ser Leu Leu Leu Gly Val Phe Gly Thr Gly Ile Ala
        275                 280                 285
Ser Ala Ser Gln Phe Leu Arg Ser Trp Leu Asn His Pro Asp Ile Ile
    290                 295                 300
Gly Tyr Ile Val Asn Gly Val Gly Val Val Trp Gln Cys His Arg Val
305                 310                 315                 320
Asn Val Thr Phe Met Ala Trp Asn Glu Ser Thr Tyr Tyr Pro Pro Val
                325                 330                 335
Asp Tyr Asn Gly Arg Lys Tyr Phe Leu Asn Asp Glu Gly Arg Leu Gln
            340                 345                 350
Thr Asn Thr Pro Glu Ala Arg Pro Gly Leu Lys Arg Val Met Trp Phe
        355                 360                 365
Gly Arg Tyr Phe Leu Gly Thr Val Gly Ser Gly Val Lys Pro Arg Arg
    370                 375                 380
Ile Arg Tyr Asn Lys Thr Ser His Asp Tyr His Leu Glu Glu Phe Glu
385                 390                 395                 400
Ala Ser Leu Asn Met Thr Pro Gln Thr Ser Ile Ala Ser Gly His Glu
                405                 410                 415
Thr Asp Pro Ile Asn His Ala Tyr Gly Thr Gln Ala Asp Leu Leu Pro
            420                 425                 430
Tyr Thr Arg Ser Ser Asn Ile Thr Ser Thr Asp Thr Gly Ser Gly Trp
        435                 440                 445
Val His Ile Gly Leu Pro Ser Phe Ala Phe Leu Asn Pro Leu Gly Trp
    450                 455                 460
Leu Arg Asp Leu Leu Ala Trp Ala Ala Trp Leu Gly Gly Val Leu Tyr
465                 470                 475                 480
Leu Ile Ser Leu Cys Val Ser Leu Pro Ala Ser Phe Ala Arg Arg Arg
                485                 490                 495
Arg Leu Gly Arg Trp Gln Glu
            500

<210> 9
<211> 424
<212> PRT
<213> Borna disease virus

<220>
<223> BoDV-1-GP p57 (gp84), aa 23-446

<400> 9
Phe Asp Leu Gln Gly Leu Ser Cys Asn Thr Asp Ser Thr Pro Gly Leu
1               5                   10                  15
Ile Asp Leu Glu Ile Arg Arg Leu Cys His Thr Pro Thr Glu Asn Val

20                  25                  30
Ile Ser Cys Glu Val Ser Tyr Leu Asn His Thr Thr Ile Ser Leu Pro
        35                  40                  45
Ala Val His Thr Ser Cys Leu Lys Tyr His Cys Lys Thr Tyr Trp Gly
    50                  55                  60
Phe Phe Gly Ser Tyr Ser Ala Asp Arg Ile Ile Asn Arg Tyr Thr Gly
65                  70                  75                  80
Thr Val Lys Gly Cys Leu Asn Asn Ser Ala Pro Glu Asp Pro Phe Glu
                85                  90                  95
Cys Asn Trp Phe Tyr Cys Cys Ser Ala Ile Thr Thr Glu Ile Cys Arg
            100                 105                 110
Cys Ser Ile Thr Asn Val Thr Val Ala Val Gln Thr Phe Pro Pro Phe
        115                 120                 125
Met Tyr Cys Ser Phe Ala Asp Cys Ser Thr Val Ser Gln Gln Glu Leu
    130                 135                 140
Glu Ser Gly Lys Ala Met Leu Ser Asp Gly Ser Thr Leu Thr Tyr Thr
145                 150                 155                 160
Pro Tyr Ile Leu Gln Ser Glu Val Val Asn Lys Thr Leu Asn Gly Thr
                165                 170                 175
Ile Leu Cys Asn Ser Ser Ser Lys Ile Val Ser Phe Asp Glu Phe Arg
            180                 185                 190
Arg Ser Tyr Ser Leu Thr Asn Gly Ser Tyr Gln Ser Ser Ser Ile Asn
        195                 200                 205
Val Thr Cys Ala Asn Tyr Thr Ser Ser Cys Arg Pro Arg Leu Lys Arg
    210                 215                 220
Arg Arg Arg Asp Thr Gln Gln Ile Glu Tyr Leu Val His Lys Leu Arg
225                 230                 235                 240
Pro Thr Leu Lys Asp Ala Trp Glu Asp Cys Glu Ile Leu Gln Ser Leu
                245                 250                 255
Leu Leu Gly Val Phe Gly Thr Gly Ile Ala Ser Ala Ser Gln Phe Leu
            260                 265                 270
Arg Ser Trp Leu Asn His Pro Asp Ile Ile Gly Tyr Ile Val Asn Gly
        275                 280                 285
Val Gly Val Val Trp Gln Cys His Arg Val Asn Val Thr Phe Met Ala
    290                 295                 300
Trp Asn Glu Ser Thr Tyr Tyr Pro Pro Val Asp Tyr Asn Gly Arg Lys
305                 310                 315                 320
Tyr Phe Leu Asn Asp Glu Gly Arg Leu Gln Thr Asn Thr Pro Glu Ala
                325                 330                 335
Arg Pro Gly Leu Lys Arg Val Met Trp Phe Gly Arg Tyr Phe Leu Gly
            340                 345                 350
Thr Val Gly Ser Gly Val Lys Pro Arg Arg Ile Arg Tyr Asn Lys Thr
        355                 360                 365
Ser His Asp Tyr His Leu Glu Glu Phe Glu Ala Ser Leu Asn Met Thr
    370                 375                 380
Pro Gln Thr Ser Ile Ala Ser Gly His Glu Thr Asp Pro Ile Asn His
385                 390                 395                 400
Ala Tyr Gly Thr Gln Ala Asp Leu Leu Pro Tyr Thr Arg Ser Ser Asn
                405                 410                 415
Ile Thr Ser Thr Asp Thr Gly Ser
            420

<210>  10
<211>  427
<212>  PRT
<213>  Artificial Sequence

<220>
<223>  BoDV-1_Phosphoprotein (aa1-201) with GST fusion expressed from
       pGEX-4T-2_BoDV-1_Phosphoprotein (aa1-201) with GST fusion

<400>  10

Met Ser Pro Ile Leu Gly Tyr Trp Lys Ile Lys Gly Leu Val Gln Pro
1               5                   10                  15
Thr Arg Leu Leu Leu Glu Tyr Leu Glu Glu Lys Tyr Glu Glu His Leu
            20                  25                  30
Tyr Glu Arg Asp Glu Gly Asp Lys Trp Arg Asn Lys Lys Phe Glu Leu
        35                  40                  45
Gly Leu Glu Phe Pro Asn Leu Pro Tyr Tyr Ile Asp Gly Asp Val Lys
    50                  55                  60
Leu Thr Gln Ser Met Ala Ile Ile Arg Tyr Ile Ala Asp Lys His Asn
65                  70                  75                  80
Met Leu Gly Gly Cys Pro Lys Glu Arg Ala Glu Ile Ser Met Leu Glu
                85                  90                  95
Gly Ala Val Leu Asp Ile Arg Tyr Gly Val Ser Arg Ile Ala Tyr Ser
            100                 105                 110
Lys Asp Phe Glu Thr Leu Lys Val Asp Phe Leu Ser Lys Leu Pro Glu
        115                 120                 125
Met Leu Lys Met Phe Glu Asp Arg Leu Cys His Lys Thr Tyr Leu Asn
    130                 135                 140
Gly Asp His Val Thr His Pro Asp Phe Met Leu Tyr Asp Ala Leu Asp
145                 150                 155                 160
Val Val Leu Tyr Met Asp Pro Met Cys Leu Asp Ala Phe Pro Lys Leu
                165                 170                 175
Val Cys Phe Lys Lys Arg Ile Glu Ala Ile Pro Gln Ile Asp Lys Tyr
            180                 185                 190
Leu Lys Ser Ser Lys Tyr Ile Ala Trp Pro Leu Gln Gly Trp Gln Ala
        195                 200                 205
Thr Phe Gly Gly Gly Asp His Pro Pro Lys Ser Asp Leu Val Pro Arg
    210                 215                 220
Gly Ser Met Ala Thr Arg Pro Ser Ser Leu Val Asp Ser Leu Glu Asp
225                 230                 235                 240
Glu Glu Asp Pro Gln Thr Leu Arg Arg Glu Arg Pro Gly Ser Pro Arg
                245                 250                 255
Pro Arg Lys Val Pro Arg Asn Ala Leu Thr Gln Pro Val Asp Gln Leu
            260                 265                 270
Leu Lys Asp Leu Arg Lys Asn Pro Ser Met Ile Ser Asp Pro Asp Gln
        275                 280                 285
Arg Thr Gly Arg Glu Gln Leu Ser Asn Asp Glu Leu Ile Lys Lys Leu
    290                 295                 300
Val Thr Glu Leu Ala Glu Asn Ser Met Ile Glu Ala Glu Glu Val Arg
305                 310                 315                 320
Gly Thr Leu Gly Asp Ile Ser Ala Arg Ile Glu Ala Gly Phe Glu Ser
                325                 330                 335
Leu Ser Ala Leu Gln Val Glu Thr Ile Gln Thr Ala Gln Arg Cys Asp
            340                 345                 350
His Ser Asp Ser Ile Arg Ile Leu Gly Glu Asn Ile Lys Ile Leu Asp
        355                 360                 365
Arg Ser Met Lys Thr Met Met Glu Thr Met Lys Leu Met Met Glu Lys
    370                 375                 380
Val Asp Leu Leu Tyr Ala Ser Thr Ala Val Gly Thr Ser Ala Pro Met
385                 390                 395                 400
Leu Pro Ser His Pro Ala Pro Pro Arg Ile Tyr Pro Gln Leu Pro Ser
                405                 410                 415
Ala Pro Thr Thr Asp Glu Trp Asp Ile Ile Pro
            420                 425

<210> 11
<211> 201
<212> PRT
<213> Borna disease virus

<220>
<223> BoDV-1-P, aa 1-201

<400> 11
Met Ala Thr Arg Pro Ser Ser Leu Val Asp Ser Leu Glu Asp Glu Glu
1               5                   10                  15
Asp Pro Gln Thr Leu Arg Arg Glu Arg Pro Gly Ser Pro Arg Pro Arg
            20                  25                  30
Lys Val Pro Arg Asn Ala Leu Thr Gln Pro Val Asp Gln Leu Leu Lys
        35                  40                  45
Asp Leu Arg Lys Asn Pro Ser Met Ile Ser Asp Pro Asp Gln Arg Thr
    50                  55                  60
Gly Arg Glu Gln Leu Ser Asn Asp Glu Leu Ile Lys Lys Leu Val Thr
65                  70                  75                  80
Glu Leu Ala Glu Asn Ser Met Ile Glu Ala Glu Glu Val Arg Gly Thr
                85                  90                  95
Leu Gly Asp Ile Ser Ala Arg Ile Glu Ala Gly Phe Glu Ser Leu Ser
            100                 105                 110
Ala Leu Gln Val Glu Thr Ile Gln Thr Ala Gln Arg Cys Asp His Ser
        115                 120                 125
Asp Ser Ile Arg Ile Leu Gly Glu Asn Ile Lys Ile Leu Asp Arg Ser
    130                 135                 140
Met Lys Thr Met Met Glu Thr Met Lys Leu Met Met Glu Lys Val Asp
145                 150                 155                 160
Leu Leu Tyr Ala Ser Thr Ala Val Gly Thr Ser Ala Pro Met Leu Pro
                165                 170                 175
Ser His Pro Ala Pro Pro Arg Ile Tyr Pro Gln Leu Pro Ser Ala Pro
            180                 185                 190
Thr Thr Asp Glu Trp Asp Ile Ile Pro
        195                 200

<210> 12
<211> 404
<212> PRT
<213> Artificial Sequence

<220>
<223> BoDV-1_Nucleoprotein (aa1-370) with N-terminal 6xHis-tag
      expressed from pET28a_BoDV-1_Nucleoprotein (aa1-370) with
      N-terminal 6xHis-tag

<400> 12
Met Gly Ser Ser His His His His His His Ser Ser Gly Leu Val Pro
1               5                   10                  15
Arg Gly Ser His Met Ala Ser Met Thr Gly Gly Gln Gln Met Gly Arg
            20                  25                  30
Gly Ser Met Pro Pro Lys Arg Arg Leu Val Asp Asp Ala Asp Ala Met
        35                  40                  45
Glu Asp Gln Asp Leu Tyr Glu Pro Pro Ala Ser Leu Pro Lys Leu Pro
    50                  55                  60
Gly Lys Phe Leu Gln Tyr Thr Val Gly Gly Ser Asp Pro His Pro Gly
65                  70                  75                  80
Ile Gly His Glu Lys Asp Ile Arg Gln Asn Ala Val Ala Leu Leu Asp
                85                  90                  95
Gln Ser Arg Arg Asp Met Phe His Thr Val Thr Pro Ser Leu Val Phe
            100                 105                 110
Leu Cys Leu Leu Ile Pro Gly Leu His Ala Ala Phe Val His Gly Gly
        115                 120                 125
Val Pro Arg Glu Ser Tyr Leu Ser Thr Pro Val Thr Arg Gly Glu Gln
    130                 135                 140
Thr Val Val Lys Thr Ala Lys Phe Tyr Gly Glu Lys Thr Thr Gln Arg
145                 150                 155                 160
Asp Leu Thr Glu Leu Glu Ile Ser Ser Ile Phe Ser His Cys Cys Ser
                165                 170                 175

Leu Leu Ile Gly Val Val Ile Gly Ser Ser Ser Lys Ile Lys Ala Gly
            180                 185                 190
Ala Glu Gln Ile Lys Lys Arg Phe Lys Thr Met Met Ala Ala Leu Asn
        195                 200                 205
Arg Pro Ser His Gly Glu Thr Ala Thr Leu Leu Gln Met Phe Asn Pro
    210                 215                 220
His Glu Ala Ile Asp Trp Ile Asn Gly Gln Pro Trp Val Gly Ser Phe
225                 230                 235                 240
Val Leu Ser Leu Leu Thr Thr Asp Phe Glu Ser Pro Gly Lys Glu Phe
                245                 250                 255
Met Asp Gln Ile Lys Leu Val Ala Ser Tyr Ala Gln Met Thr Thr Tyr
            260                 265                 270
Thr Thr Ile Lys Glu Tyr Leu Ala Glu Cys Met Asp Ala Thr Leu Thr
        275                 280                 285
Ile Pro Val Val Ala Tyr Glu Ile Arg Asp Phe Leu Glu Val Ser Ala
    290                 295                 300
Lys Leu Lys Glu Asp His Ala Asp Leu Phe Pro Phe Leu Gly Ala Ile
305                 310                 315                 320
Arg His Pro Asp Ala Ile Lys Leu Ala Pro Arg Ser Phe Pro Asn Leu
                325                 330                 335
Ala Ser Ala Ala Phe Tyr Trp Ser Lys Lys Glu Asn Pro Thr Met Ala
            340                 345                 350
Gly Tyr Arg Ala Ser Thr Ile Gln Pro Gly Ala Ser Val Lys Glu Thr
        355                 360                 365
Gln Leu Ala Arg Tyr Arg Arg Arg Glu Ile Ser Arg Gly Glu Asp Gly
    370                 375                 380
Ala Glu Leu Ser Gly Glu Ile Ser Ala Ile Met Lys Met Ile Gly Val
385                 390                 395                 400
Thr Gly Leu Asn


<210> 13
<211> 370
<212> PRT
<213> Borna disease virus


<220>
<223> BoDV-1-N, aa 1-370

<400> 13
Met Pro Pro Lys Arg Arg Leu Val Asp Asp Ala Asp Ala Met Glu Asp
1               5                   10                  15
Gln Asp Leu Tyr Glu Pro Pro Ala Ser Leu Pro Lys Leu Pro Gly Lys
            20                  25                  30
Phe Leu Gln Tyr Thr Val Gly Gly Ser Asp Pro His Pro Gly Ile Gly
        35                  40                  45
His Glu Lys Asp Ile Arg Gln Asn Ala Val Ala Leu Leu Asp Gln Ser
    50                  55                  60
Arg Arg Asp Met Phe His Thr Val Thr Pro Ser Leu Val Phe Leu Cys
65                  70                  75                  80
Leu Leu Ile Pro Gly Leu His Ala Ala Phe Val His Gly Gly Val Pro
                85                  90                  95
Arg Glu Ser Tyr Leu Ser Thr Pro Val Thr Arg Gly Glu Gln Thr Val
            100                 105                 110
Val Lys Thr Ala Lys Phe Tyr Gly Glu Lys Thr Thr Gln Arg Asp Leu
        115                 120                 125
Thr Glu Leu Glu Ile Ser Ser Ile Phe Ser His Cys Cys Ser Leu Leu
    130                 135                 140
Ile Gly Val Val Ile Gly Ser Ser Ser Lys Ile Lys Ala Gly Ala Glu
145                 150                 155                 160
Gln Ile Lys Lys Arg Phe Lys Thr Met Met Ala Ala Leu Asn Arg Pro
                165                 170                 175

Ser His Gly Glu Thr Ala Thr Leu Leu Gln Met Phe Asn Pro His Glu
            180                 185                 190
Ala Ile Asp Trp Ile Asn Gly Gln Pro Trp Val Gly Ser Phe Val Leu
        195                 200                 205
Ser Leu Leu Thr Thr Asp Phe Glu Ser Pro Gly Lys Glu Phe Met Asp
    210                 215                 220
Gln Ile Lys Leu Val Ala Ser Tyr Ala Gln Met Thr Thr Tyr Thr Thr
225                 230                 235                 240
Ile Lys Glu Tyr Leu Ala Glu Cys Met Asp Ala Thr Leu Thr Ile Pro
                245                 250                 255
Val Val Ala Tyr Glu Ile Arg Asp Phe Leu Glu Val Ser Ala Lys Leu
            260                 265                 270
Lys Glu Asp His Ala Asp Leu Phe Pro Phe Leu Gly Ala Ile Arg His
        275                 280                 285
Pro Asp Ala Ile Lys Leu Ala Pro Arg Ser Phe Pro Asn Leu Ala Ser
    290                 295                 300
Ala Ala Phe Tyr Trp Ser Lys Lys Glu Asn Pro Thr Met Ala Gly Tyr
305                 310                 315                 320
Arg Ala Ser Thr Ile Gln Pro Gly Ala Ser Val Lys Glu Thr Gln Leu
                325                 330                 335
Ala Arg Tyr Arg Arg Arg Glu Ile Ser Arg Gly Glu Asp Gly Ala Glu
            340                 345                 350
Leu Ser Gly Glu Ile Ser Ala Ile Met Lys Met Ile Gly Val Thr Gly
        355                 360                 365
Leu Asn
    370

<210> 14
<211> 249
<212> PRT
<213> Borna disease virus

<220>
<223> BoDV-1-GP, gp56, aa 1-249 of p57

<400> 14
Met Gln Pro Ser Met Ser Phe Leu Ile Gly Phe Gly Thr Leu Val Leu
1               5                   10                  15
Val Leu Ser Ala Arg Thr Phe Asp Leu Gln Gly Leu Ser Cys Asn Thr
            20                  25                  30
Asp Ser Thr Pro Gly Leu Ile Asp Leu Glu Ile Arg Arg Leu Cys His
        35                  40                  45
Thr Pro Thr Glu Asn Val Ile Ser Cys Glu Val Ser Tyr Leu Asn His
    50                  55                  60
Thr Thr Ile Ser Leu Pro Ala Val His Thr Ser Cys Leu Lys Tyr His
65                  70                  75                  80
Cys Lys Thr Tyr Trp Gly Phe Phe Gly Ser Tyr Ser Ala Asp Arg Ile
                85                  90                  95
Ile Asn Arg Tyr Thr Gly Thr Val Lys Gly Cys Leu Asn Asn Ser Ala
            100                 105                 110
Pro Glu Asp Pro Phe Glu Cys Asn Trp Phe Tyr Cys Cys Ser Ala Ile
        115                 120                 125
Thr Thr Glu Ile Cys Arg Cys Ser Ile Thr Asn Val Thr Val Ala Val
    130                 135                 140
Gln Thr Phe Pro Pro Phe Met Tyr Cys Ser Phe Ala Asp Cys Ser Thr
145                 150                 155                 160
Val Ser Gln Gln Glu Leu Glu Ser Gly Lys Ala Met Leu Ser Asp Gly
                165                 170                 175
Ser Thr Leu Thr Tyr Thr Pro Tyr Ile Leu Gln Ser Glu Val Val Asn
            180                 185                 190
Lys Thr Leu Asn Gly Thr Ile Leu Cys Asn Ser Ser Ser Lys Ile Val
        195                 200                 205

Ser Phe Asp Glu Phe Arg Arg Ser Tyr Ser Leu Thr Asn Gly Ser Tyr
    210                 215                 220
Gln Ser Ser Ser Ile Asn Val Thr Cys Ala Asn Tyr Thr Ser Ser Cys
225                 230                 235                 240
Arg Pro Arg Leu Lys Arg Arg Arg Arg
                245

<210> 15
<211> 254
<212> PRT
<213> Borna disease virus

<220>
<223> BoDV-1-GP, gp43, aa 250-503 of p57

<400> 15
Asp Thr Gln Gln Ile Glu Tyr Leu Val His Lys Leu Arg Pro Thr Leu
1               5                   10                  15
Lys Asp Ala Trp Glu Asp Cys Glu Ile Leu Gln Ser Leu Leu Leu Gly
            20                  25                  30
Val Phe Gly Thr Gly Ile Ala Ser Ala Ser Gln Phe Leu Arg Ser Trp
        35                  40                  45
Leu Asn His Pro Asp Ile Ile Gly Tyr Ile Val Asn Gly Val Gly Val
    50                  55                  60
Val Trp Gln Cys His Arg Val Asn Val Thr Phe Met Ala Trp Asn Glu
65                  70                  75                  80
Ser Thr Tyr Tyr Pro Pro Val Asp Tyr Asn Gly Arg Lys Tyr Phe Leu
                85                  90                  95
Asn Asp Glu Gly Arg Leu Gln Thr Asn Thr Pro Glu Ala Arg Pro Gly
            100                 105                 110
Leu Lys Arg Val Met Trp Phe Gly Arg Tyr Phe Leu Gly Thr Val Gly
        115                 120                 125
Ser Gly Val Lys Pro Arg Arg Ile Arg Tyr Asn Lys Thr Ser His Asp
    130                 135                 140
Tyr His Leu Glu Glu Phe Glu Ala Ser Leu Asn Met Thr Pro Gln Thr
145                 150                 155                 160
Ser Ile Ala Ser Gly His Glu Thr Asp Pro Ile Asn His Ala Tyr Gly
                165                 170                 175
Thr Gln Ala Asp Leu Leu Pro Tyr Thr Arg Ser Ser Asn Ile Thr Ser
            180                 185                 190
Thr Asp Thr Gly Ser Gly Trp Val His Ile Gly Leu Pro Ser Phe Ala
        195                 200                 205
Phe Leu Asn Pro Leu Gly Trp Leu Arg Asp Leu Leu Ala Trp Ala Ala
    210                 215                 220
Trp Leu Gly Gly Val Leu Tyr Leu Ile Ser Leu Cys Val Ser Leu Pro
225                 230                 235                 240
Ala Ser Phe Ala Arg Arg Arg Arg Leu Gly Arg Trp Gln Glu
                245                 250

<210> 16
<211> 227
<212> PRT
<213> Borna disease virus

<220>
<223> BoDV-1-GP, gp56, aa 23-249 of p57

<400> 16
Phe Asp Leu Gln Gly Leu Ser Cys Asn Thr Asp Ser Thr Pro Gly Leu
1               5                   10                  15
Ile Asp Leu Glu Ile Arg Arg Leu Cys His Thr Pro Thr Glu Asn Val

20                  25                  30
Ile Ser Cys Glu Val Ser Tyr Leu Asn His Thr Thr Ile Ser Leu Pro
        35                  40                  45
Ala Val His Thr Ser Cys Leu Lys Tyr His Cys Lys Thr Tyr Trp Gly
    50                  55                  60
Phe Phe Gly Ser Tyr Ser Ala Asp Arg Ile Ile Asn Arg Tyr Thr Gly
65                  70                  75                  80
Thr Val Lys Gly Cys Leu Asn Asn Ser Ala Pro Glu Asp Pro Phe Glu
                85                  90                  95
Cys Asn Trp Phe Tyr Cys Cys Ser Ala Ile Thr Thr Glu Ile Cys Arg
            100                 105                 110
Cys Ser Ile Thr Asn Val Thr Val Ala Val Gln Thr Phe Pro Pro Phe
        115                 120                 125
Met Tyr Cys Ser Phe Ala Asp Cys Ser Thr Val Ser Gln Gln Glu Leu
    130                 135                 140
Glu Ser Gly Lys Ala Met Leu Ser Asp Gly Ser Thr Leu Thr Tyr Thr
145                 150                 155                 160
Pro Tyr Ile Leu Gln Ser Glu Val Val Asn Lys Thr Leu Asn Gly Thr
                165                 170                 175
Ile Leu Cys Asn Ser Ser Ser Lys Ile Val Ser Phe Asp Glu Phe Arg
            180                 185                 190
Arg Ser Tyr Ser Leu Thr Asn Gly Ser Tyr Gln Ser Ser Ser Ile Asn
        195                 200                 205
Val Thr Cys Ala Asn Tyr Thr Ser Ser Cys Arg Pro Arg Leu Lys Arg
    210                 215                 220
Arg Arg Arg
225

<210> 17
<211> 197
<212> PRT
<213> Borna disease virus


<220>
<223> BoDV-1-GP, gp43, aa 250-446 of p57

<400> 17
Asp Thr Gln Gln Ile Glu Tyr Leu Val His Lys Leu Arg Pro Thr Leu
1               5                   10                  15
Lys Asp Ala Trp Glu Asp Cys Glu Ile Leu Gln Ser Leu Leu Leu Gly
            20                  25                  30
Val Phe Gly Thr Gly Ile Ala Ser Ala Ser Gln Phe Leu Arg Ser Trp
        35                  40                  45
Leu Asn His Pro Asp Ile Ile Gly Tyr Ile Val Asn Gly Val Gly Val
    50                  55                  60
Val Trp Gln Cys His Arg Val Asn Val Thr Phe Met Ala Trp Asn Glu
65                  70                  75                  80
Ser Thr Tyr Tyr Pro Pro Val Asp Tyr Asn Gly Arg Lys Tyr Phe Leu
                85                  90                  95
Asn Asp Glu Gly Arg Leu Gln Thr Asn Thr Pro Glu Ala Arg Pro Gly
            100                 105                 110
Leu Lys Arg Val Met Trp Phe Gly Arg Tyr Phe Leu Gly Thr Val Gly
        115                 120                 125
Ser Gly Val Lys Pro Arg Arg Ile Arg Tyr Asn Lys Thr Ser His Asp
    130                 135                 140
Tyr His Leu Glu Glu Phe Glu Ala Ser Leu Asn Met Thr Pro Gln Thr
145                 150                 155                 160
Ser Ile Ala Ser Gly His Glu Thr Asp Pro Ile Asn His Ala Tyr Gly
                165                 170                 175
Thr Gln Ala Asp Leu Leu Pro Tyr Thr Arg Ser Ser Asn Ile Thr Ser
            180                 185                 190
Thr Asp Thr Gly Ser

        195

<210> 18
<211> 1383
<212> DNA
<213> Artificial Sequence

<220>
<223> Coding sequence of pLEXSY-sat2.1_BoDV-1.gp_aa23-446

<400> 18
atggcctcga ggctcgtccg tgtgctggcc gccgccatgc tggttgcagc ggccgtgtcg      60

gtcgacgctg gcgcctctct agacttcgat ctgcagggcc tgagctgcaa cacagactct     120

acaccgggcc tgatcgacct tgagattcgc cgtctgtgcc acacgccgac cgagaacgtg     180

atctcttgcg aggtgtcgta cctgaaccac acgacgatta gcctgccggc ggtgcataca     240

agctgcctga agtaccactg caagacgtac tggggcttct tcggcagcta cagcgcggac     300

cgcatcatca accgttacac gggcacagtg aagggctgcc tcaacaactc tgcaccagag     360

gacccgttcg agtgcaactg gttctactgc tgcagcgcga tcacgaccga gatttgccgt     420

tgcagcatca cgaacgtgac ggtggcggtg cagacgttcc cgccgtttat gtactgcagc     480

ttcgcggact gcagcacggt gtctcagcag gagcttgaga gcggcaaggc aatgcttagc     540

gacggcagca cgctgacgta cacgccgtac attcttcaga gcgaggtggt caacaagacg     600

ctgaacggca cgatcctgtg caacagcagc agcaagatcg tgtcgttcga cgagttccgc     660

cgcagctact ctctgacgaa cggttcctac cagagcagct cgatcaacgt gacctgcgcg     720

aactacacga gcagctgccg tccacgtctt aagcgccgtc gtcgtgacac gcagcagatc     780

gagtacctgg tgcacaagct gcgcccgaca ctgaaggatg catgggagga ctgcgagatc     840

ctgcagtctc tgctgctcgg cgtgttcggt acaggcattg caagcgcgag ccagtttctg     900

cgcagctggc tgaaccatcc ggacatcatc ggctacatcg tgaacggcgt gggtgtcgtg     960

tggcagtgcc atcgtgtgaa cgtgacattc atggcgtgga acgagagcac gtactacccg    1020

ccggtggatt acaacggccg caagtacttc ctgaacgacg agggtcgcct gcagaccaac    1080

acaccagagg cacgcccagg ccttaagcgc gtgatgtggt tcggccgcta cttcctgggt    1140

acagtcggtt ctggtgtcaa gccacgccgc atccgctaca acaagaccag ccacgactac    1200

caccttgagg agttcgaggc gagcctgaac atgacgccgc agacttctat tgcgagcggc    1260

cacgagacag acccgatcaa ccatgcatac ggcacacagg cagacctgct gccatacacg    1320

cgtagcagca acatcacgag cacagacact ggtagcggta cccaccacca tcaccaccac    1380

tag                                                                  1383

<210> 19
<211> 1284

<212> DNA
<213> Artificial Sequence

<220>
<223> pGEX-4T-2_BoDV-1_Phosphoprotein (aa1-201) with GST fusion

<400> 19
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa     420

acatatttaa atggtgatca tgtaacccat cctgacttca tgttgtatga cgctcttgat     480

gttgttttat acatggaccc aatgtgcctg gatgcgttcc caaaattagt ttgttttaaa     540

aaacgtattg aagctatccc acaaattgat aagtacttga aatccagcaa gtatatagca     600

tggcctttgc agggctggca agccacgttt ggtggtggcg accatcctcc aaaatcggat     660

ctggttccgc gtggatccat ggcaacgcga ccatcgagtc tggtggactc cctggaggac     720

gaagaagatc cccagacact acgacgggaa cgaccggggt caccaagacc acggaaggtc     780

ccaaggaatg cattgaccca accagtagac cagctcctga aggacctcag gaagaacccc     840

tccatgatct cagacccaga ccagcgaacc ggaagggagc agctgtcgaa tgatgagcta     900

atcaagaagt tagtgacgga gctggccgag aatagcatga tcgaggctga ggaggtgcgg     960

ggcactcttg gagacatctc ggctcgtatc gaggcagggt ttgagtccct gtccgccctc    1020

caagtggaaa ccatccagac agctcagcgg tgcgatcact ccgacagcat cagaatcctc    1080

ggcgagaaca tcaagatact agatcgctcc atgaagacaa tgatggagac aatgaagctc    1140

atgatggaga aggtggatct cctctacgca tcaaccgccg ttgggacctc tgcacccatg    1200

ttgccctccc atcctgcacc tccgcgcatt tatccccagc tcccaagtgc cccgacaacg    1260

gatgaatggg acatcatacc ataa                                           1284

<210> 20
<211> 1215
<212> DNA
<213> Artificial Sequence

<220>
<223> pET28a_BoDV-1_Nucleoprotein (aa1-370) with N-terminal 6xHis-tag

<400> 20

atgggcagca gccatcatca tcatcatcac agcagcggcc tggtgccgcg cggcagccat 60

atggctagca tgactggtgg acagcaaatg ggtcgcggat ccatgccacc caagagacgc 120

ctggttgatg acgccgatgc catggaggat caagatctat atgaaccccc agcgagcctc 180

cctaagctcc ctgggaaatt cctacaatac accgttgggg ggtctgaccc gcatccgggt 240

atagggcatg agaaagacat caggcagaac gcagtggcat tgttagacca gtcacggcgc 300

gatatgtttc acacagtaac gcctagcctt gtgtttctat gtttgctaat cccaggactg 360

cacgctgcgt ttgttcacgg aggggtgcct cgtgaatcct acctatcgac gcctgtcacg 420

cgtggagaac agactgttgt taagactgcg aagttttacg gggaaaagac gacgcagcgt 480

gatctcaccg agctggagat ctcctctatc ttcagccatt gttgctcatt actaataggg 540

gttgtgatag gatcgtcgtc taagatcaaa gcaggagccg agcagatcaa gaaaaggttt 600

aaaactatga tggcagcctt aaaccggcca tcccatggtg agactgctac actactccag 660

atgtttaatc cacatgaggc tatagattgg attaacggcc aaccctgggt aggctccttt 720

gtgttgtctc tactaactac agactttgag tccccaggta aagaatttat ggaccagatt 780

aagcttgtcg caagttatgc acagatgact acgtacacta ctataaagga gtacctcgca 840

gaatgcatgg atgctaccct tacaatcccc gtagttgcct atgagatccg tgacttttta 900

gaagtttcag caaagcttaa ggaggatcat gctgacctgt tcccgtttct gggggccatt 960

agacaccccg acgctatcaa gctggcgcca cgaagctttc ccaatctggc ctccgcagcg 1020

ttttactgga gtaagaagga aaaccccaca atggcaggct accgggcctc caccatccag 1080

ccgggcgcaa gtgtcaagga aacccagctt gcccggtata ggcgccgcga gatatctcgt 1140

ggagaggacg gggcagagct ctcaggtgag atctctgcca taatgaagat gataggtgtg 1200

actggtctaa actaa 1215

## Description

The invention relates to a solid phase comprising one or more immobilized Borna disease virus (BoDV) antigens for the detection of BoDV specific antibodies in a sample, wherein said one or more immobilized BoDV antigens comprise BoDV-glycoprotein (BoDV-GP) and/or fragments thereof. In embodiments, the solid phase additionally comprises at least one antigen selected from the group comprising BoDV-N-protein, BoDV-P-protein and BoDV-X-protein, and/or fragments thereof, wherein preferably each antigen is located on a spatially separated area of the solid surface enabling individual and/or independent detection of antibodies directed against each antigen. The invention further relates to a method and kit for the detection of BoDV specific antibodies and/or BoDV infection.

### BACKGROUND OF THE INVENTION

Borna disease virus (BoDV) is a member of the genus Orthobornavirus, family Bornaviridae, order Mononegavirales. It has a nonsegmented, minus-sense genome of 8.9kb, containing, by one definition, five genes. Three transcription initiation and four, possibly five, transcription termination signals have been identified in the viral genome. It has been suggested that the genome includes at least six open reading frames: specifically, 3'-N-P/X-M-GP-L-5'. The major structural proteins include the nucleoprotein (N), the phosphoprotein (P), the matrix protein (M), the glycoprotein (GP), and the RNA-dependent RNA polymerase (L) protein; a nonstructural small polypeptide (X/p10) that is abundantly expressed in infected cells is absent in virus particles.

A broad range of cell types can be infected with Borna disease virus, including neurons and glial cells derived from several animal species. Natural infection with Borna virus is described predominantly in horses and sheep in areas of central Europe, but cases have also been reported in other species. The route of natural Borna disease virus infection of horses is uncertain, although oronasal transmission has been proposed based on results from experimental infections of laboratory animals.

The virus can establish a chronic infection characterized by neurotropism and low production of virus. Infection may be asymptomatic or may result in disease characterized by movement and behavioral abnormalities. Furthermore, BoDV has been reported to cause meningoencephalitis and encephalomyelitis primarily in horses in Europe, but also in sheep, cattle, and a variety of other domestic and wild animal species. On rare occasions, cats and dogs are affected.

Naturally, infected horses exhibiting such abnormalities usually recover, but the disease may progress to paralysis and death. Experimentally infected rats and primates also exhibit behavioral abnormalities. Because of these effects on other animals, several recent studies have tried to determine if the virus is associated with neurological disease in man. Fewer than half of affected cats test positive for BDV-specific antibodies, and PCR has not proved to be a reliable test in this species. The disease can be definitively diagnosed only through postmortem examination.

Hoffmann et al. (A variegated squirrel bornavirus associated with fatal human encephalitis, N Engl J Med 2015; 373, pages 154-162) describe a terminal virus infection of a plurality of older male human patients having pre-existing conditions (high blood pressure, diabetes and/or obesity) with a bornavirus which affects variegated squirrels ("VSBV-1", i.e. Variegated Squirrel Bornavirus-1). All the patients suffered from a thrombosis, which led to pulmonary embolism. The patients suffered from encephalitis, which however was not a limbic encephalitis, but a syndrome with oedematous lesions in the cerebral cortical regions without limbic distribution. An indirect immunofluorescence test (IIFT) based on a feline cell line infected with the complete BoDV-1 virus was used to detect antibodies against bornaviruses. Furthermore, other ELISA based methods for detecting antibodies against BoDV-P, BoDV-N or BoDV-X have been suggested in the art in order to detect BoDV-specific antibodies in patient sample. However, such systems are not reliably leading to the identifications of BoDV infected subjects.

In light of the prior art there remains a need in the art for alternative and improved means for detecting BoDV-specific antibodies. Such systems can be particularly useful for the identification of BoDV-infected subjects, which are not identified by the methods presently available, or which are only identified by more complicated methods in comparison to routine methods employing solid phases comprising coupled antigens or antibodies.

### SUMMARY OF THE INVENTION

In light of the prior art the technical problem underlying the present invention is to provide alternative and/or improved means that enable detection of BoDV specific antibodies in a routine assay.

This problem is solved by the features of the independent claims. Preferred embodiments of the present invention are provided by the dependent claims.

The invention therefore relates to a solid phase comprising one or more immobilized Borna disease virus (BoDV) antigens for the detection of BoDV specific antibodies in a sample, wherein said one or more immobilized BoDV antigens comprise BoDV-glycoprotein (BoDV-GP) and/or fragments thereof.

The present invention is based on the entirely surprising finding that it is possible to detect BoDV-specific antibodies with high accuracy in samples form infected subjects by using BoDV-GP or fragments thereof that is couple to a solid phase. Tests using the solid phase of the invention are surprisingly advantageous in comparison to known solid phase comprising other BoDV proteins, such as N or P, but not GP. It appears that the presence of GP-specific antibodies is a superior marker in comparison to the presence of antibodies against other BoDV proteins in order to identify subjects that have encountered or are infected with BoDV. However, so far, no functional ELISA or other serological test directed to the specific detection of anti-BoDV-GP antibodies in a sample of a subject or patient has been developed or reported. The present invention is based on the finding that using solid phases with immobilized BoDV-GP provide reliable information about the current or previous presence of BoDV in a subject or patient, which is entirely surprising since all previous serological tests were directed to the detection of BoDV-antibodies without distinguishing between the different antigens, or were directed to BoDV-N, BoDV-P and/or BoDV-X.

In embodiments of the invention, the solid phase comprises additionally at least one antigen selected from the group comprising BoDV-N-protein, BoDV-P-protein and BoDV-X-protein, and/or fragments thereof. Such embodiments have of the solid phase of the invention have the advantage that it is possible to simultaneously also detect antibodies to other proteins of BoDV that may be present in a test-sample, which can lead to a higher accuracy of a test using the solid phase of the invention.

In a preferred embodiment, what is differentiated is whether the patient to be diagnosed has antibodies against one or more than one, preferably two or more or three or more different BoDV antigens.

In embodiments, of the invention, each antigen is located on spatially separated area of the solid surface enabling individual and/or independent detection of antibodies directed against each antigen. It is a particular advantage of such embodiments that it is possible to differentially detect the presence of antibodies against the different antigens present on the solid phase. Such information, which for example indicate the presence of BoDV-GP specific antibodies, but the absence of BoDV-N specific antibodies can be useful in order to draw further conclusion about the infective state or course of disease of the subject, which was the donor of the tested sample.

A spatially separated area of the solid phase can be a defined area of a well of a microtiter plate, wherein for example within one well there are multiple defined areas, such as a spot, that comprise only one specific immobilized antigen. Accordingly, such a well can comprise several spots of different immobilized antigens, and detection of a signal in a specific spot indicates the presence of antibodies directed against the respective antigen.

In embodiments of the invention, the solid phase is flat or spherical, such as a microtiter plate, one or multiple beads, a glass slide or a membrane, and/or comprises or consists of plastic, glass, nitrocellulose, PVDF, polystyrene or latex. In embodiments, the solid surface is the surface of a test strip of a line assay or lateral flow assay.

For example, the solid phase of the invention can be a surface of a microtiter plate, preferably the surface of a well of a microtiter plate, which is preferably flat. Such a microtiter plate can be made of different materials suitable for coupling antigens, which are routinely used in ELISA-based methods known to a skilled person, such as tissue culture plastic materials, in particular polystyrene, or glass.

In further embodiments, the solid phase of the invention can be the surface of one or multiple spherical beads. In such embodiments, the term "a solid surface" can refer to the overall surfaces of multiple beads. In embodiments, the solid surfaces are more than one kind of beads, for example beads of different size, density or labeling. In embodiments, each kind of antigen is coupled to a different kind of bead. For example, BoDV-GP is coupled to a first kind of bead, BoDV-N is coupled to a second kind of bead, and BoDV-P is coupled to a third kind of bead. In such embodiments, the surface of each kind of bead is understood as representing a spatially separated area or the overall solid surface. The beads of the invention can be made of any material known to a skilled person as being routinely used for coupling proteins and in particular antibodies or antigens, for example for use in a serological detection method. Such beads can be made of latex or plastic or glass or other materials known in the art.

In further embodiments, the solid surface of the invention can be a membrane, such as a PVDF or nitrocellulose membrane. Such a membrane can also be used for coating a surface, for example of a well of a microtiter plate or the surface of a test strip of a line assay or lateral flow assay.

In embodiments, the BoDV is selected from the group comprising BoDV-1, BoDV-2 and VSBV-1. The present invention invention relates to the detection of antibodies of antigens of different kinds of BoDV. As used herein, the term "Borna disease viruses (BoDV)" relates to *Bornaviridae* in general, which is a family of viruses in the order *Mononegavirales.* In embodiments, the BoDV is a *Orthobornavirus.* In further embodiments, the BoDV is a *mammalian orthobornavirus.* In specific embodiments, the BoDV is *mammalian 1 orthobornavirus,* which comprises BoDV-1 and BoDV-2. In further embodiments, the BoDV is *mammalian 2 orthobornavirus,* which comprises in particular variegated squirrel bornavirus 1 (VSBV-1).

The preferable viruses comprised by this general term that is subject of the present invention are BoDV-1, BoDV-2 and VSBV-1.

Currently 18 viruses are assigned to ten species included in two genera in this family. The two genera are *Carboviruses* and *Orthobornaviruses.*

Accordingly, in embodiments of the invention the BoDV-GP coupled to a solid phase is the GP from BoDV-1. In embodiments, the BoDV-GP coupled to a solid phase is the GP from BoDV-2. In embodiments, the BoDV-GP coupled to a solid phase is the GP from VSBV-1.

In embodiments, the BoDV-GP or fragments thereof coupled to the solid surface is glycosylated. It is particular advantage of the present invention that it is possible to couple a glycosylated form of the BoDV-GP to the solid surface of the invention. On the surface of the virion, GP is present in glycosylated form and therefore a host generates antibodies against the glycosylated protein present on the virion. Accordingly, it is possible that certain antibodies only bind to glycosylated GP, but not to unglycosylated GP.

Glycosylation of GP can occur through various techniques known to a skilled person. For example, GP can be expressed in a eukaryotic expression system. Expression of GP by using a *L. tarentolae* expression system is particularly advantageous, since *Trypanosomatidae,* which comprise a *L. tarentolae,* are rich in glycoproteins with a pattern of glycosylation closely related to those in mammals and higher vertebrates. Thus, one of the main advantages of a *L. tarentolae* expression system is the mammalian-type posttranslational modification of target proteins.

In embodiments of the invention, the BoDV-GP or fragments thereof is present as a dimer consisting of BoDV-GP fragments gp43 and gp56 or fragments thereof.

In embodiments, the dimers are covalently or non-covalently linked to each other. Covalent linkage of the fragments can be through at least one disulfide bond between amino acid residues of the fragments.

In embodiments, each of the fragments can be coupled to the solid surface. In embodiments, only one of the fragments of the dimer is coupled to the solid surface and the other fragment is bound to the coupled fragment. In embodiments, gp43 and gp56 or fragments thereof are linked by at least one disulfide bond.

In embodiments, gp43 and gp56 are immobilized on the same area of the solid phase. In further embodiment, gp43 and gp56 are immobilized on spatially separated areas of the solid surface enabling individual and/or independent detection of antibodies directed against each of the BoDV-GP fragments.

As used herein the term "gp43" relates to the c-terminal fragment of BoDV-GP that is generated by cellular furin cleavage and may also be termed GP-C. "gp56" relates to the aminoterminal fragment of BoDV-GP that is generated by cellular furin cleavage and may also be termed GP-N. Glycosylation of BoDV-GP occurs predominantly in the amino-terminal gp56 fragment.

In embodiments, the solid phase of the invention the one or more immobilized antigen comprises gp56. In particular embodiments, gp56 is glycosylated. In embodiments, the solid surface comprises immobilized gp43, which may be glycosylated. In embodiments, the solid surface comprises immobilized gp43 and gp56, wherein preferably at least gp56 is glycosylated.

In embodiments, the one or more immobilized antigens are recombinant proteins.

In embodiments, the BoDV-GP is isolated from a eukaryotic protein expression system, preferably a *Leishmania tarentolae* expression system. The same can be the case for one or more or all of the potential other BoDV antigens that may be immobilized on the solid phase of the invention. In embodiments the eukaryotic protein expression system is an insect cell expression system.

In further embodiments, the BoDV-GP is isolated from a bacaterial expression system, such as preferably an *E.coli* expression system.

In embodiments of the invention, BoDV-GP is a dimer of gp43 and gp56 or fragments thereof, wherein gp43 and gp56 have preferably been generated by cleavage from a longer precursor BoDV-GP, such as for example full length BoDV-GP, in a cell of an expression system, such as in a eukaryotic cell of a eukaryotic expression system. In such embodiments, intracellular cleavage can occur by a furin enzyme. In alternative embodiments, cleavage of the longer precursor may occur after purification of the precursor from the expression system. Alternatively, gp43 and gp56 may have been expressed individually in a suitable expression system disclosed herein and/or an expression system known to a person skilled in the art and dimerization may have occurred after individual purification of gp43 and gp56.

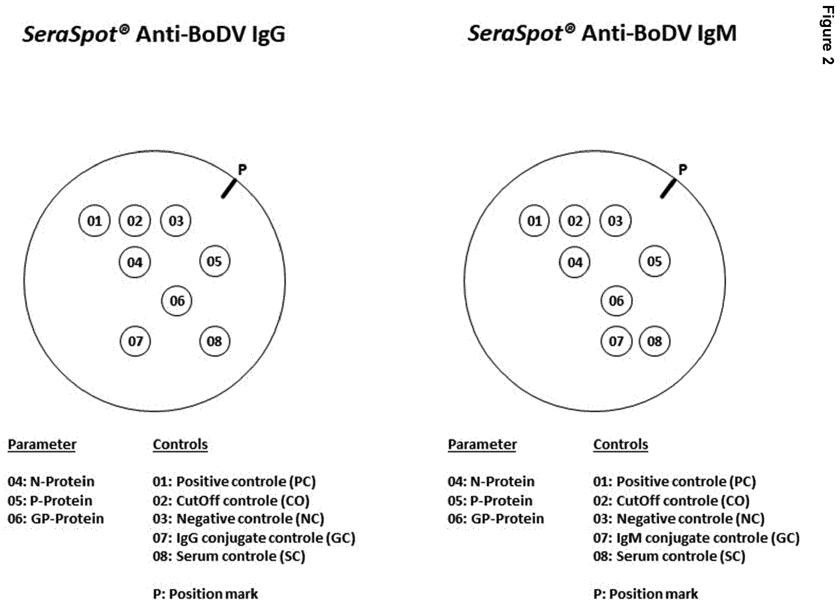

In a specific embodiment of the solid surface of the invention, the BoDV-GP and at least one of BoDV-N-protein, BoDV-P-protein and BoDV-X-protein are immobilized on spatially separated areas of a surface of a well of a microtiter plate, and wherein said surface comprises additional spatially separated areas serving as controls, for example for binding of labeled secondary affinity reagents and functionality of means for detecting a signal emitted from a labeled secondary affinity reagent. A preferred version of such an embodiment of the invention is depicted in Figure 2 of this application. However, each kind of control area and antigen area depicted in Figure 2 can also be used individually in the context of further embodiments of the invention, without leading to an intermediate generalization.

In embodiments of the invention, the solid phase is a SeraSpot® plate. A SeraSpot® is an array-format based spot immunoassay (SIA), preferably in 96well-microtitration plates, which can be used in particular for the detection of IgG and/or IgM antibodies directed against specific antigens, such as the BoDV antigens of the invention. Test specific controls are integrated in every array: A cut-off control, a negative control, and a sample control. Latter indicates the presence / absence of serum. Results are calculated in ratios via the staining intensities of the specific antigen spots relative to the cut-off spot. Bound antibodies from samples can be detected by HRP-labeled anti-human IgG / IgM and chromogenic precipitating substrate solution. Immune complexes can be visualized as pale blue to dark blue spots. Developed arrays can scanned with Seramun SpotSight® plate scanner and evaluated by software Seramun SpotSight® scan.

In preferred embodiments of the invention, detection is performed by Horseradish peroxidase-(HRP)-labelled detector molecules and a HRP-catalysed substrate reaction. At the site of reaction blue spots are developed by precipitated product from colorless substrate solution. Color intensity is correlated to the analyte concentration. Pale blue to dark blue spots are visible by eye. This technology is commonly used in SeraSpot® assay, which are well described in the art and can be applied to various embodiments of the invention.

The present invention further relates to an in vitro method for the detection of a BoDV antibodies in a sample, comprising providing a sample, which has preferably been isolated from a subject,
- providing a solid phase of the invention comprising one or more immobilized BoDV antigens,
- contacting said sample with said solid phase, and
- detecting antibodies from said sample that bind to said one or more BoDV antigens.

Furthermore, the present invention relates to an in vitro method for the detection of a BoDV infection in a subject, comprising providing a sample of said subject,
- providing a solid phase of the invention comprising one or more immobilized BoDV antigens,
- contacting said sample with said solid phase, and
- detecting antibodies from said sample that bind to said one or more BoDV antigens.

In embodiments of the invention, the subject is human. In further embodiment, the subject may be an animal, such as a horse, dog, cat, sheep, cattle, a domestic animal, or a wild animal. The person skilled in the art is aware of animal species that can be infected or are suspected of being infected by BoDV.

In embodiments, the method of the invention can be used
- for the diagnosis of a neurological condition, encephalitis, limbic encephalitis, PNS, encephalomyelitis, leukoencephalopathy, retinitis and/or optic atrophy,
- for predicting neurological post-transplant complications or for screening organ donors or donor organs or blood donors, and/or
- for differentiating an autoimmune and an infection-related encephalitis.

Furthermore, the present invention relates to an isolated BoDV-GP or fragments thereof, wherein the BoDV-GP has been purified from a *Leishmania tarentolae* expression system. The invention is based on the surprising finding that purification of BoDV-GP from an eukaryotic expression system and in particular from *L. tarentolae* results in a GP protein or fragment thereof that is more specifically recognized by BoDV-specific antibodies present in a subject that is or has been infected with BoDV. Accordingly, detection methods using such an isolated BoDV-GP or fragment thereof, which may be immobilized on a solid phase, have better specificity and sensitivity as compared to previously described methods of the state of the art. Furthermore, the use of such islolated proteins makes it possible to provide a routine laboratory assay that can be performed for example in a standard ELISA-like method without the need of using for example living cells.

In embodiments, the invention relates to an isolated BoDV-GP or fragments thereof, wherein the BoDV-GP has been purified from a *Leishmania tarentolae* expression system and is a dimer consisting of BoDV-GP fragments gp43 and gp56 or fragments thereof. The use of this eukaryotic expression system is advantageous since it is possible to produce a BoDV-GP dimer with a structure that is very closely related to the structure of BoDV-GP as expressed in infected cells and incorporated into BoDV viral particles. Accordingly, the structure of the isolated GP-protein of the invention is very similar to the structure of the GP-protein present in the body of an infected subject and against which the subject generates specific antibodies. Therefore, these antibodes that are present in sample of such a subject will more efficiently and specifically bind to GP-proteins of the invention as compared to previously reported isolated GP-proteins.

In further embodiments, the invention relates to an isolated BoDV-GP or fragments thereof, wherein the BoDV-GP has been purified from a *Leishmania tarentolae* expression system and is a dimer consisting of BoDV-GP fragments gp43 and gp56 or fragments thereof, wherein gp43 and gp56 or fragments thereof are linked by at least one disulfide bond.

Furthermore, the invention also relates to a nucleic acid molecule encoding a BoDV-GP protein for expression and isolation from an expression system, such as *Leishmania tarentolae.* The invention can additionally comprise nucleic acids for expression of additional BoDV proteins, such as N or P. In embodiments, these additional BoDV proteins are encoded by nucleic acid molecules suitable for expression of the proteins in a eukaryotic or prokaryotic expression system.

Additionally, the present invention relates a kit for the detection of BoDV specific antibodies in a sample, comprising
- a solid phase of the present invention, or
- one or more isolated BoDV antigens comprising BoDV-GP or fragments thereof and preferably at least one or BoDV-N-protein, BoDV-P-protein and BoDV-X-protein, or fragments thereof, and a solid phase for immobilization of said one or more isolated BoDV antigens, and
- means for detecting antibodies bound to BoDV antigens immobilized on the solid surface, such as labeled secondary affinity reagents directed against the bound antibodies and means for detecting a signal emitted from said label, and optionally,
- computer software configured for determining the presence and/or the amount of bound antibodies.

In embodiments, the kit comprises a an isolated BoDV-GP or fragments thereof of, wherein the BoDV-GP has been purified from a *Leishmania tarentolae* expression system, and preferably is a dimer consisting of BoDV-GP fragments gp43 and gp56 or fragments thereof, wherein gp43 and gp56 or fragments thereof are possibly linked by at least one disulfide bond.

In embodiments, the kit of the inventio comprises the computer software as an obligatory feature.

In embodiments, the invention also relates to system for the detection of BoDV specific antibodies in a sample, comprising a kit of the invention as disclosed herein, and a computer system for automated analysis of one or more samples, comprising a computer processing device and a plate reader or camera device suitable for detecting the signal of the means for detecting antibodies bound to BoDV antigens immobilized on the solid surface, such as labelled (secondary) affinity reagents.

All features disclosed herein in the context of the solid phase of the invention are herewith also disclosed in the context of the methods of the invention as well as in the context of the isolated BoDV-GP or the kit of the invention. The other way around, features that have only been disclosed in the context of a method of the invention, the kit of the invention or the isolated BoDV-GP of the invention can also relate to all other aspects of the invention, such as the solid phase.

### DETAILED DESCRIPTION OF THE INVENTION

All cited documents of the patent and non-patent literature are hereby incorporated by reference in their entirety.

The invention relates to a solid phase comprising one or more immobilized Borna disease virus (BoDV) antigens for the detection of BoDV specific antibodies in a sample, wherein said one or more immobilized BoDV antigens comprise BoDV-glycoprotein (BoDV-GP) and/or fragments thereof.

As used herein, Borna disease virus (BoDV) relates to to *Bornaviridae* in general, which is a family of viruses in the order *Mononegavirales.* They are Enveloped, spherical, and have a diameter from about 70 to 130 nm. The family of *Bornaviridae comprises two genera, Carboviruses and Orthobornaviruses.*

*Carboviruses* comprise the species Queensland carbovirus (jungle carpet python virus (JCPV)) and *Southwest carbovirus* (southwest carpet python virus (SWCPV)). *Orthobornaviruses* comprise the species *Elapid 1 orthobornavirus* (Loveridge's garter snake virus 1 (LGSV-1)), *Mammalian 1 orthobornavirus* (Borna disease virus 1 (BoDV-1) and Borna disease virus 2 (BoDV-2)), *Mammalian 2 orthobornavirus* (variegated squirrel bornavirus 1 (VSBV-1)), *Passeriform 1 orthobornavirus* (canary bornavirus 1 (CnBV-1), canary bornavirus 2 (CnBV-2), canary bornavirus 3 (CnBV-3)), *Passeriform 2 orthobornavirus* (estrildid finch bornavirus 1 (EsBV-*1)), Psittaciform 1 orthobornavirus* (parrot bornavirus 1 (PaBV-1), parrot bornavirus 2 (PaBV-2), parrot bornavirus 3 (PaBV-3), parrot bornavirus 4 (PaBV-4), parrot bornavirus 7 (PaBV-7)), *Psittaciform 2 orthobornavirus* (parrot bornavirus 5 (PaBV-5)), and *Waterbird 1 orthobornavirus* (aquatic bird bornavirus 1 (ABBV-1), aquatic bird bornavirus 2 (ABBV-2)).

In embodiments, the BoDV of the invention is a *mammalian orthobornavirus.* In specific embodiments, the BoDV is *mammalian 1 orthobornavirus,* which comprises BoDV-1 and BoDV-2. In further embodiments, the BoDV is *mammalian 2 orthobornavirus,* which comprises variegated squirrel bornavirus 1 (VSBV-1).

The Borna disease viruses 1 and 2 (BoDV-1 and BoDV-2) are members of the species Mammalian 1 orthobornavirus and cause Borna disease in mammals. Infection with these viruses causes Borna disease, also known as sad horse disease, which is an infectious neurological syndrome of warm-blooded animals. BoDV-1 and 2 cause abnormal behaviour and fatality. Borna disease viruses 1 and 2 are neurotropic viruses. Although Borna disease viruses 1 and 2 are mainly seen as the causative agent of Borna disease in horses and other animals, they are also controversially discussed as human infectious agents and therefore as potential zoonotic agents.

Since behavioral disease has been studied in BoDV-1 infected animals like rhesus monkeys, tree shrews, and rats, BoDV-1 has also been hypothesized to be associated with humans psychiatric conditions such as schizophrenia and affective psychoses.

The role of BoDV-1 and -2 in human illness is controversial and it is yet to be established whether BoDV-1 or -2 cause any overt disease in humans.

Between 2011 and 2013, three German breeders of variegated squirrels (Sciurus variegatoides) had encephalitis with similar clinical signs and died 2 to 4 months after onset of the clinical symptoms. Genomic analysis found a previously unknown orthobornavirus in a contact squirrel and in brain tissue from the three men, the researchers reported, and it is the "likely causative agent" in their deaths. Prior to this, bornaviruses were not thought to be responsible for human diseases (Hoffmann et al. NEJM 373(2): 154-162).

Orthobornavirions are enveloped, with spherical geometries and helical capsids. The diameter is around 70 to 130 nm. Genomes are linear, around 8.9 kb in length. The genome codes for up to 9 proteins, wherein the most relevant are the following: BoDV-N (Helical nucleoside protein), BoDV-GP (Envelope glycoprotein), BoDV-L (Viral polymerase), BoDV-P (Phosphoprotein involved in replication), BoDV-M (Matrix protein) and BoDV-X (Has not been fully characterized but is probably involved in nuclear transport).

As used herein, all proteins expressed in an infected subject and in particular those that are present in part of the virions, and fragments of such proteins are considered BoDV antigens. Such fragments of BoDV proteins should be big or long enough to serve as an antibody-epitope. Accordingly, in particular the BoDV-N, BoDV-GP, BoDV-M, BoDV-X and BoDV-L and fragments of each of these proteins of the different BoDV disclosed herein can serve as antigens in the context of the present invention. All BoDV and the aa sequences of BoDV proteins and antigens can be identified by whole genome sequencing using the methodology as described in Shahhosseini N et al. (2017; Infect Genet Evol.; 55:260-268), in combination with the Tappe D et al. (2018; Emerg Infect Dis. 2018 Jun;24(6):978-987).

Antigens of the invention comprise, without limitation, BoDV-1-N, BoDV-1-GP, BoDV-1-M, BoDV-1-X, BoDV-1-L, BoDV-2-N, BoDV-2-GP, BoDV-2-M, BoDV-2-X, BoDV-2-L, VSBV-1-N, VSBV-1-GP, VSBV-1-M, VSBV-1-X and VSBV-1-L.

In the context of the invention, the BoDV antigens or fragments thereof can be recombinant proteins or animal-derived proteins Recombinant proteins are biotechnologically produced proteins that have been created with the help of genetically modified organisms or with transiently transfected cell cultures. By means of genetic engineering, a large number of bacterial organisms, fungi or mammalian cells can be used for the production of foreign proteins. Preferred systems for such production are the intestinal bacterium Escherichia coli, various yeast species or cell lines of insect or mammalian cells.

In most cases, the genetic information for the protein is cloned into a vector, e.g. a plasmid, which is then transformed or transfected into the host organism. Protein engineering can be used to adapt the properties of the recombinant protein. Vector design allows the vector to be adapted. After cloning a transgene (insert) into a vector, the recombinant DNA produced is introduced into an organism. Occasionally a clone is isolated as a genetically identical starting point for a cell culture by limiting dilution cloning. Reporter genes are occasionally used to facilitate the identification of a transgenic clone. The subsequent overexpression of the recombinant protein allows higher yields than those found in the original organism. Selection markers allow the selective growth of only the transgenic organisms. For proteins that are toxic to their expression organism, inducible promoters are used that allow the cells to grow until gene expression is induced. After a growth phase, the toxic genes are induced, the cells are "harvested" and digested by adding protease inhibitors. In the course of protein purification, the desired protein is separated from cellular proteins and other biomolecules and subsequently characterized. Often proteolytic protein tags are used for purification and detection, which are then separated.

A system used for the genetically engineered production of proteins should preferably be able to grow rapidly in large fermenters at the lowest possible cost; should produce the desired substances efficiently with the correct posttranslational modifications and; should possibly secrete them into the culture medium. In the context of the invention, production of the BoDV antigens and in particular of BoDV-GP, which comprises posttranslational modifications comprising furin cleavage and glycosylation, should preferably occur in a eukaryotic expression system capable of performing such modification. In the context of the invention, expression of BoDV-GP in a *L. tarentolae* expression system turned out to be particularly suited.

In embodiments of the invention, BoDV antigens and in particular BODV-GP is generated through expression in a eukaryotic expression system. Eukaryotic expression systems are frequently employed for the production of recombinant proteins as therapeutics as well as research and diagnostic tools. Most commonly used expression systems are based on stably transfected adherent CHO cells or nonadherent lymphoid cell lines. An efficient alternative is the infection of insect cells by recombinant baculoviruses. Transient expression in mammalian cells, e.g., COS cells, is often used for the production of smaller quantities of proteins. The choice of a suitable expression system depends largely on the biochemical and biological properties of the protein of interest, as well as on the nature of the planned experiments or applications of the protein and the amount of recombinant protein required.

*Leishmania tarentolae* is a preferred expression system of the invention for the expression and isolation of BoDV antigens and in particular BoDV-GP. As explained herein, a variety of recombinant protein expression systems have been developed for heterologous genes in both prokaryotic and eukaryotic systems such as bacteria, yeast, mammals, insects, transgenic animals, and plants. *Leishmania tarentolae* is a trypanosomatid protozoan parasite of the white-spotted wall gecko (*Tarentola annularis*) and has been suggested as candidate for heterologous genes expression. Trypanosomatidae such as *Leishmania tarentolae* are rich in glycoproteins, which can account for more than 10% of total protein; the oligosaccharide structures are similar to those of mammals with N-linked galactose, and fucose residues, and the system has been successfully used for expression of cytoplasmic enzymes and membrane receptors.

Six major ORFs for BoDV proteins have been found in the BoDV genome sequence. These ORFs code for polypeptides with predicted size of 40 kDa (p40, BoDV-N), 24 kDa (p24, BoDV-P), 10 kDa (p10, BoDV-X), 16 kDa (p16, BoDV-M), 56 kDa (p56, BoDV-GP) and 180 kDa (p180 BoDV-L), respectively. Based on their positions in the viral genome and abundance in infected cells and virion particles, together with their biochemical and sequence features, p40, p24 and p16 BoDV polypeptides correspond to the viral nucleoprotein (N), the phosphoprotein (P) transcriptional activator, and matrix (M) proteins, respectively.

Two isoforms of the BoDV N (p39 and p38) are found in BoDV-infected cells. These two forms of the viral N appear to be encoded by two different mRNA species. Differential usage of two in-frame initiation codons present in the BoDV p40 gene may also contribute to the production of BoDV p39/38. BoDV p39 contains both a nuclear localization signal (NLS) and a nuclear export signal (NES), whereas p38 harbors only the NES.

BoDV p24 is an acidic polypeptide (predicted I.P. of 4.8), that has a high Ser-Thr content (16%), with phosphorylation at serine residues which is mediated by both protein kinase C and casein kinase II. These features are consistent with those of the phosphoprotein (P) transcriptional activator found in other negative sense ssRNA viruses. BoDV p24 contains a bipartite NLS in the sequence. In addition to P, a 16 kDa polypeptide (P') is also translated from the second in-frame AUG codon in the P ORF.

BoDV-X, p10, encodes a polypeptide of 10 kDa present in BoDV-infected cells. BoDV X starts within the same mRNA transcription unit, 46 nt upstream from p24 and overlaps, in a different frame, with the 71 N-terminal aa of p24. Recent study indicates that BoDV X harbors a NLS in the N-terminus of the sequence.

BoDV p16, the BoDV-M protein, is a non-glycosylated M protein, associated at the inner surface of the viral membrane.

BoDV-GP is encoded by BoDV ORF4 (p56), which overlaps, in a different frame, with the C-terminus of ORF p16, and is capable of encoding a 503 aa polypeptide with a predicted size of 56 kDa. Based on its sequence features, BoDV p56 is the counterpart of the virus surface glycoproteins (GP) found in other negative sense ssRNA viruses. The p56 gene directs the synthesis of three glycosylated polypeptides of about 84 or 94 kDa (GP-84/94, G), 43 kDa (gp43, GP-C) and 45 to 55 kDa (GP-N, gp56). BoDV-GP corresponds to the full length of the p56 gene, whereas gp43/GP-C and gp56/GP-N represent the C-terminal subunit and the N-terminal subunit of ORF p56, respectively. Both GP-C and GP-N are associated with BoDV infectious particles. BoDV p56 gene products have been suggested to play an important role in the early steps of BoDV infection.

BoDV ORF5 (p180) is capable of encoding a polypetide with a predicted size of 180 kDa, whose deduced aa sequence displays strong homology to other negative sense ssRNA virus polymerases, members of the L protein family. An additional ORF predicted in mRNA species generated via RNA splicing would encode a variant BoDV-L with a predicted size of 190 kDa (BVp190). BoDV p190 corresponds to BoDV p180 with 153 aa added to its N-terminus. Recent evidence suggests that p190, rather than p180, is the active BDV L. BDV L contains the NLS in the sequence.

The present invention surprisingly found that the use of BoDV-GP or fragments thereof, such as gp43 and gp56 or fragments thereof, and also dimers of gp43 and gp56 or fragments thereof, are particularly advantageous for detecting BoDV specific antibodies in a sample. Furthermore, detection of BoDV-GP specific antibodies is particularly advantageous in methods of detecting a BoDV infection in a subject, who is providing the sample for testing the presence of the BoDV specific antibodies. These advantageous are probably due to the exposed location of BoDV-GP on the surface of the virion. However, since BoDV-GP is highly glycosylated in its native location on the viral particle, the advantages of using BoDV-GP in the context of the invention are particularly pronounced when the protein/antigen of the invention is also glycosylated in a similar glycosylation pattern as during infection in a subject.

Glycosylation is the reaction in which a carbohydrate, i.e. a glycosyl donor, is attached to a hydroxyl or other functional group of another molecule, such as a proteins, which serves as a glycosyl acceptor. In biology, glycosylation mainly refers in particular to the enzymatic process that attaches glycans to proteins, or other organic molecules. Glycosylation is a form of co-translational and post-translational modification. Glycans serve a variety of structural and functional roles in membrane and secreted proteins. The majority of proteins synthesized in the rough endoplasmic reticulum undergoes glycosylation. It is an enzyme-directed site-specific process, as opposed to the non-enzymatic chemical reaction of glycation. Glycosylation profoundly affects biological activity, function, clearance from circulation, and crucially, antigenicity. The cells of nonhuman species do not glycosylate their proteins in the same way as human cells do. In many cases, the differences are profound. Accordingly, it is hard to predict which expression system for recombinant glycoproteins will result in suitable posttranslational modification and in particular glycosylation patterns that are useful for the intended use of the expressed proteins. In the context of the invention, it turned out that expression of BoDV-GP in *Leishmania tarentolae* results in a particularly suitable glycosylation that enables detection of BoDV specific antibodies with high specificity and sensitivity.

Preferred amino acid sequences of antigens, proteins or polypeptides of embodiments of the present invention are disclosed in *Table* 1. Preferred nucleic acid sequences comprised by nucleic acid molecules encoding for BoDV antigens and proteins of the invention are disclosed in *Table 2.*

**Table 1: Amino Acid sequences of BoDV protein of the invention.**

| SEQ ID NO. | Amino Acid Sequence | Description |
|---|---|---|
| SEQ ID NO: 7 | MASRLVRVLAAAMLVAAAVSVDAGASLDFDLQGLS CNTDSTPGLIDLEIRRLCHTPTENVISCEVSYLNHTT ISLPAVHTSCLKYHCKTYWGFFGSYSADRIINRYTG TVKGCLNNSAPEDPFECNWFYCCSAITTEICRCSIT NVTVAVQTFPPFMYCSFADCSTVSQQELESGKAM LSDGSTLTYTPYILQSEVVNKTLNGTILCNSSSKIVS FDEFRRSYSLTNGSYQSSSINVTCANYTSSCRPRL KRRRRDTQQIEYLVHKLRPTLKDAWEDCEILQSLLL GVFGTGIASASQFLRSWLNHPDIIGYIVNGVGVVW QCHRVNVTFMAWNESTYYPPVDYNGRKYFLNDEG RLQTNTPEARPGLKRVMWFGRYFLGTVGSGVKPR RIRYNKTSHDYHLEEFEASLNMTPQTSIASGHETDP INHAYGTQADLLPYTRSSNITSTDTGSGTHHHHHH | BoDV-1 .gp_aa23-446 construct expressed from pLEXSY-sat2.1_BoDV-1.gp_aa23-446 Underlined: amino acid (aa) 23-446 of BoDV-1-GP; **Underlined bold:** furin cleavage site; bold: c-terminal HIS-tag; *italics:* aa encoded by vector DNA (incl. aa encoded by multiple cloning site). |
| SEQ ID NO: 8 | MQPSMSFLIGFGTLVLVLSARTFDLQGLSCNTDSTP GLIDLEIRRLCHTPTENVISCEVSYLNHTTISLPAVHT SCLKYHCKTYWGFFGSYSADRIINRYTGTVKGCLN NSAPEDPFECNWFYCCSAITTEICRCSITNVTVAVQ TFPPFMYCSFADCSTVSQQELESGKAMLSDGSTLT YTPYILQSEVVNKTLNGTILCNSSSKIVSFDEFRRSY SLTNGSYQSSSINVTCANYTSSCRPRLKRRRRDTQ QIEYLVHKLRPTLKDAWEDCEILQSLLLGVFGTGIAS | BoDV-1-GP p57 aa 1-503 |
| | ASQFLRSWLNHPDIIGYIVNGVGVVWQCHRVNVTF MAWNESTYYPPVDYNGRKYFLNDEGRLQTNTPEA RPGLKRVMWFGRYFLGTVGSGVKPRRIRYNKTSH DYHLEEFEASLNMTPQTSIASGHETDPINHAYGTQA DLLPYTRSSNITSTDTGSGWVHIGLPSFAFLNPLGW LRDLLAWAAWLGGVLYLISLCVSLPASFARRRRLG RWQE | |
| SEQ ID NO: 9 | FDLQGLSCNTDSTPGLIDLEIRRLCHTPTENVISCEV SYLNHTTISLPAVHTSCLKYHCKTYWGFFGSYSAD RIINRYTGTVKGCLNNSAPEDPFECNWFYCCSAITT EICRCSITNVTVAVQTFPPFMYCSFADCSTVSQQEL ESGKAMLSDGSTLTYTPYILQSEVVNKTLNGTILCN SSSKIVSFDEFRRSYSLTNGSYQSSSINVTCANYTS SCRPRLKRRRRDTQQIEYLVHKLRPTLKDAWEDCE ILQSLLLGVFGTGIASASQFLRSWLNHPDIIGYIVNG VGVVWQCHRVNVTFMAWNESTYYPPVDYNGRKY FLNDEGRLQTNTPEARPGLKRVMWFGRYFLGTVG SGVKPRRIRYNKTSHDYHLEEFEASLNMTPQTSIAS GHETDPINHAYGTQADLLPYTRSSNITSTDTGS | BoDV-1-GP p57 (gp84), aa 23-446 |
| SEQ ID NO: 10 | MSPILGYWKIKGLVQPTRLLLEYLEEKYEEHLYER DEGDKWRNKKFELGLEFPNLPYYIDGDVKLTQSM AIIRYIADKHNMLGGCPKERAEISMLEGAVLDIRYG VSRIAYSKDFETLKVDFLSKLPEMLKMFEDRLCHK TYLNGDHVTHPDFMLYDALDVVLYMDPMCLDAFP KLVCFKKRIEAIPQIDKYLKSSKYIAWPLQGWQAT FGGGDHPPKSDLVPRGSMATRPSSLVDSLEDEED PQTLRRERPGSPRPRKVPRNALTQPVDQLLKDLRK NPSMISDPDQRTGREQLSNDELIKKLVTELAENSMI EAEEVRGTLGDISARIEAGFESLSALQVETIQTAQR CDHSDSIRILGENIKILDRSMKTMMETMKLMMEKVD LLYASTAVGTSAPMLPSHPAPPRIYPQLPSAPTTDE WDIIP | BoDV-1_Phosphoprotein (aa1-201) with GST fusion expressed from pGEX-4T-2_BoDV-1_Phosphoprotein (aa1-201) with GST fusion Underlined: amino acid (aa) 1-201 of BoDV-1-P; **bold:** GST tag; *italics:* aa encoded by vector DNA incl. multiple cloning site. |
| SEQ ID NO: 11 | MATRPSSLVDSLEDEEDPQTLRRERPGSPRPRKV PRNALTQPVDQLLKDLRKNPSMISDPDQRTGREQL SNDELIKKLVTELAENSMIEAEEVRGTLGDISARIEA GFESLSALQVETIQTAQRCDHSDSIRILGENIKILDR SMKTMMETMKLMMEKVDLLYASTAVGTSAPMLPS HPAPPRIYPQLPSAPTTDEWDIIP | BoDV-1-P aa 1-201 |
| SEQ ID NO: 12 | MGSSHHHHHHSSGLVPRGSHMASMTGGQQMGR GSMPPKRRLVDDADAMEDQDLYEPPASLPKLPGK FLQYTVGGSDPHPGIGHEKDIRQNAVALLDQSRRD MFHTVTPSLVFLCLLIPGLHAAFVHGGVPRESYLST PVTRGEQTVVKTAKFYGEKTTQRDLTELEISSIFSH CCSLLIGVVIGSSSKIKAGAEQIKKRFKTMMAALNRP SHGETATLLQMFNPHEAIDWINGQPWVGSFVLSLL TTDFESPGKEFMDQIKLVASYAQMTTYTTIKEYLAE CMDATLTIPVVAYEIRDFLEVSAKLKEDHADLFPFLG AIRHPDAIKLAPRSFPNLASAAFYWSKKENPTMAGY RASTIQPGASVKETQLARYRRREISRGEDGAELSG EISAIMKMIGVTGLN | BoDV-1_Nucleoprotein (aa1-370) with N-terminal 6xHis-tag expressed from pET28a_BoDV-1_Nucleoprotein (aa1-370) with N-terminal 6xHis-tag Underlined: amino acid (aa) 1-370 of BoDV-1-N; **bold:** HIS tag; *italics:* aa encoded by vector DNA incl. multiple cloning site. |
| SEQ ID NO: 13 | MPPKRRLVDDADAMEDQDLYEPPASLPKLPGKFL QYTVGGSDPHPGIGHEKDIRQNAVALLDQSRRDMF HTVTPSLVFLCLLIPGLHAAFVHGGVPRESYLSTPV TRGEQTVVKTAKFYGEKTTQRDLTELEISSIFSHCC SLLIGVVIGSSSKIKAGAEQIKKRFKTMMAALNRPSH GETATLLQMFNPHEAIDWINGQPWVGSFVLSLLTT DFESPGKEFMDQIKLVASYAQMTTYTTIKEYLAECM DATLTIPVVAYEIRDFLEVSAKLKEDHADLFPFLGAI RHPDAIKLAPRSFPNLASAAFYWSKKENPTMAGYR ASTIQPGASVKETQLARYRRREISRGEDGAELSGEI SAIMKMIGVTGLN | BoDV-1-N aa 1-370 |
| SEQ ID NO: 14 | MQPSMSFLIGFGTLVLVLSARTFDLQGLSCNTDSTP GLIDLEIRRLCHTPTENVISCEVSYLNHTTISLPAVHT SCLKYHCKTYWGFFGSYSADRIINRYTGTVKGCLN NSAPEDPFECNWFYCCSAITTEICRCSITNVTVAVQ TFPPFMYCSFADCSTVSQQELESGKAMLSDGSTLT YTPYILQSEVVNKTLNGTILCNSSSKIVSFDEFRRSY SLTNGSYQSSSINVTCANYTSSCRPRLKRRRR | BoDV-1-GP, gp56, aa 1-249 of p57 |
| SEQ ID NO: 15 | DTQQIEYLVHKLRPTLKDAWEDCEILQSLLLGVFGT GIASASQFLRSWLNHPDIIGYIVNGVGVVWQCHRV NVTFMAWNESTYYPPVDYNGRKYFLNDEGRLQTN TPEARPGLKRVMWFGRYFLGTVGSGVKPRRIRYN KTSHDYHLEEFEASLNMTPQTSIASGHETDPINHAY GTQADLLPYTRSSNITSTDTGSGWVHIGLPSFAFLN PLGWLRDLLAWAAWLGGVLYLISLCVSLPASFARR RRLGRWQE | BoDV-1-GP, gp43, aa 250-503 of p57 |
| SEQ ID NO: 16 | FDLQGLSCNTDSTPGLIDLEIRRLCHTPTENVISCEV SYLNHTTISLPAVHTSCLKYHCKTYWGFFGSYSAD RIINRYTGTVKGCLNNSAPEDPFECNWFYCCSAITT EICRCSITNVTVAVQTFPPFMYCSFADCSTVSQQEL ESGKAMLSDGSTLTYTPYILQSEVVNKTLNGTILCN SSSKIVSFDEFRRSYSLTNGSYQSSSINVTCANYTS SCRPRLKRRRR | BoDV-1-GP, gp56, aa 23-249 of p57 |
| SEQ ID NO: 17 | DTQQIEYLVHKLRPTLKDAWEDCEILQSLLLGVFGT GIASASQFLRSWLNHPDIIGYIVNGVGVVWQCHRV NVTFMAWNESTYYPPVDYNGRKYFLNDEGRLQTN TPEARPGLKRVMWFGRYFLGTVGSGVKPRRIRYN KTSHDYHLEEFEASLNMTPQTSIASGHETDPINHAY GTQADLLPYTRSSNITSTDTGS | BoDV-1-GP, gp43, aa 250-446 of p57 |

**Table 2: DNA-Sequences encoding BoDV proteins of the present invention.**

| | | |
|---|---|---|
| SEQ ID NO: 18 | ATGGCCTCGAGGCTCGTCCGTGTGCTGGCCGCC GCCATGCTGGTTGCAGCGGCCGTGTCGGTCGAC GCTGGCGCCTCTCTAGACTTCGATCTGCAGGGC CTGAGCTGCAACACAGACTCTACACCGGGCCTG ATCGACCTTGAGATTCGCCGTCTGTGCCACACG CCGACCGAGAACGTGATCTCTTGCGAGGTGTCG TACCTGAACCACACGACGATTAGCCTGCCGGCG GTGCATACAAGCTGCCTGAAGTACCACTGCAAGA CGTACTGGGGCTTCTTCGGCAGCTACAGCGCGG ACCGCATCATCAACCGTTACACGGGCACAGTGA AGGGCTGCCTCAACAACTCTGCACCAGAGGACC CGTTCGAGTGCAACTGGTTCTACTGCTGCAGCG CGATCACGACCGAGATTTGCCGTTGCAGCATCA CGAACGTGACGGTGGCGGTGCAGACGTTCCCGC CGTTTATGTACTGCAGCTTCGCGGACTGCAGCAC GGTGTCTCAGCAGGAGCTTGAGAGCGGCAAGGC AATGCTTAGCGACGGCAGCACGCTGACGTACAC GCCGTACATTCTTCAGAGCGAGGTGGTCAACAA GACGCTGAACGGCACGATCCTGTGCAACAGCAG CAGCAAGATCGTGTCGTTCGACGAGTTCCGCCG CAGCTACTCTCTGACGAACGGTTCCTACCAGAGC AGCTCGATCAACGTGACCTGCGCGAACTACACG AGCAGCTGCCGTCCACGTCTTAAGCGCCGTCGT CGTGACACGCAGCAGATCGAGTACCTGGTGCAC AAGCTGCGCCCGACACTGAAGGATGCATGGGAG GACTGCGAGATCCTGCAGTCTCTGCTGCTCGGC GTGTTCGGTACAGGCATTGCAAGCGCGAGCCAG | Coding sequence of pLEXSY-sat2.1_BoDV-1.gp_aa23-446 Underlined: cDNA coding for amino acid (aa) 23-446 of BoDV-1-GP; **Underlined bold:** furin cleavage site; bold: cDNA coding for c-terminal HIS-tag and stop codon; *italics:* coding vector DNA incl. multiple cloning site. |
| | TTTCTGCGCAGCTGGCTGAACCATCCGGACATCA TCGGCTACATCGTGAACGGCGTGGGTGTCGTGT GGCAGTGCCATCGTGTGAACGTGACATTCATGG CGTGGAACGAGAGCACGTACTACCCGCCGGTGG ATTACAACGGCCGCAAGTACTTCCTGAACGACGA GGGTCGCCTGCAGACCAACACACCAGAGGCACG CCCAGGCCTTAAGCGCGTGATGTGGTTCGGCCG CTACTTCCTGGGTACAGTCGGTTCTGGTGTCAAG CCACGCCGCATCCGCTACAACAAGACCAGCCAC GACTACCACCTTGAGGAGTTCGAGGCGAGCCTG AACATGACGCCGCAGACTTCTATTGCGAGCGGC CACGAGACAGACCCGATCAACCATGCATACGGC ACACAGGCAGACCTGCTGCCATACACGCGTAGC AGCAACATCACGAGCACAGACACTGGTAGCGGT ACCCACCACCATCACCACCACTAG | |
| SEQ ID NO: 19 | ATGTCCCCTATACTAGGTTATTGGAAAATTAAGG GCCTTGTGCAACCCACTCGACTTCTTTTGGAATA TCTTGAAGAAAAATATGAAGAGCATTTGTATGA GCGCGATGAAGGTGATAAATGGCGAAACAAAA AGTTTGAATTGGGTTTGGAGTTTCCCAATCTTCC TTATTATATTGATGGTGATGTTAAATTAACACAG TCTATGGCCATCATACGTTATATAGCTGACAAG CACAACATGTTGGGTGGTTGTCCAAAAGAGCGT GCAGAGATTTCAATGCTTGAAGGAGCGGTTTTG GATATTAGATACGGTGTTTCGAGAATTGCATATA GTAAAGACTTTGAAACTCTCAAAGTTGATTTTCT TAGCAAGCTACCTGAAATGCTGAAAATGTTCGA AGATCGTTTATGTCATAAAACATATTTAAATGGT GATCATGTAACCCATCCTGACTTCATGTTGTATG ACGCTCTTGATGTTGTTTTATACATGGACCCAAT GTGCCTGGATGCGTTCCCAAAATTAGTTTGTTTT AAAAAACGTATTGAAGCTATCCCACAAATTGAT AAGTACTTGAAATCCAGCAAGTATATAGCATGG CCTTTGCAGGGCTGGCAAGCCACGTTTGGTGGT GGCGACCATCCTCCAAAATCGGATCTGGTTCCG CGTGGATCCATGGCAACGCGACCATCGAGTCTG GTGGACTCCCTGGAGGACGAAGAAGATCCCCAG ACACTACGACGGGAACGACCGGGGTCACCAAGA CCACGGAAGGTCCCAAGGAATGCATTGACCCAA CCAGTAGACCAGCTCCTGAAGGACCTCAGGAAG AACCCCTCCATGATCTCAGACCCAGACCAGCGA ACCGGAAGGGAGCAGCTGTCGAATGATGAGCTA ATCAAGAAGTTAGTGACGGAGCTGGCCGAGAAT AGCATGATCGAGGCTGAGGAGGTGCGGGGCACT CTTGGAGACATCTCGGCTCGTATCGAGGCAGGG | pGEX-4T-2_BoDV-1_Phosphoprotein (aa1-201) with GST fusion Underlined: cDNA coding for amino acid (aa) 1-201 of BoDV-1-P including stop codon; bold: cDNA coding for GST tag; *italics:* coding vector DNA incl. multiple cloning site. |
| | TTTGAGTCCCTGTCCGCCCTCCAAGTGGAAACCA TCCAGACAGCTCAGCGGTGCGATCACTCCGACA GCATCAGAATCCTCGGCGAGAACATCAAGATACT AGATCGCTCCATGAAGACAATGATGGAGACAATG AAGCTCATGATGGAGAAGGTGGATCTCCTCTACG CATCAACCGCCGTTGGGACCTCTGCACCCATGTT GCCCTCCCATCCTGCACCTCCGCGCATTTATCCC CAGCTCCCAAGTGCCCCGACAACGGATGAATGG GACATCATACCATAA | |
| SEQ ID NO: 20 | ATGGGCAGCAGCCATCATCATCATCATCACAGC AGCGGCCTGGTGCCGCGCGGCAGCCATATGGC TAGCATGACTGGTGGACAGCAAATGGGTCGCGG ATCCATGCCACCCAAGAGACGCCTGGTTGATGA CGCCGATGCCATGGAGGATCAAGATCTATATGAA CCCCCAGCGAGCCTCCCTAAGCTCCCTGGGAAA TTCCTACAATACACCGTTGGGGGTCTGACCCG CATCCGGGTATAGGGCATGAGAAAGACATCAGG CAGAACGCAGTGGCATTGTTAGACCAGTCACGG CGCGATATGTTTCACACAGTAACGCCTAGCCTTG TGTTTCTATGTTTGCTAATCCCAGGACTGCACGC TGCGTTTGTTCACGGAGGGGTGCCTCGTGAATC CTACCTATCGACGCCTGTCACGCGTGGAGAACA GACTGTTGTTAAGACTGCGAAGTTTTACGGGGAA AAGACGACGCAGCGTGATCTCACCGAGCTGGAG ATCTCCTCTATCTTCAGCCATTGTTGCTCATTACT AATAGGGGTTGTGATAGGATCGTCGTCTAAGATC AAAGCAGGAGCCGAGCAGATCAAGAAAAGGTTT AAAACTATGATGGCAGCCTTAAACCGGCCATCCC ATGGTGAGACTGCTACACTACTCCAGATGTTTAA TCCACATGAGGCTATAGATTGGATTAACGGCCAA CCCTGGGTAGGCTCCTTTGTGTTGTCTCTACTAA CTACAGACTTTGAGTCCCCAGGTAAAGAATTTAT GGACCAGATTAAGCTTGTCGCAAGTTATGCACAG ATGACTACGTACACTACTATAAAGGAGTACCTCG CAGAATGCATGGATGCTACCCTTACAATCCCCGT AGTTGCCTATGAGATCCGTGACTTTTTAGAAGTTT CAGCAAAGCTTAAGGAGGATCATGCTGACCTGTT CCCGTTTCTGGGGGCCATTAGACACCCCGACGC TATCAAGCTGGCGCCACGAAGCTTTCCCAATCTG GCCTCCGCAGCGTTTTACTGGAGTAAGAAGGAA AACCCCACAATGGCAGGCTACCGGGCCTCCACC ATCCAGCCGGGCGCAAGTGTCAAGGAAACCCAG CTTGCCCGGTATAGGCGCCGCGAGATATCTCGT GGAGAGGACGGGGCAGAGCTCTCAGGTGAGAT | pET28a_BoDV-1_Nucleoprotein (aa1-370) with N-terminal 6xHis-tag Underlined: cDNA coding for amino acid (aa) 1-370 of BoDV-1-N including stop codon; bold: cDNA coding for N-terminal HIS tag; *italics*: coding vector DNA incl. multiple cloning site |
| | CTCTGCCATAATGAAGATGATAGGTGTGACTGGT CTAAACTAA | |

The invention further relates to functionally analogous sequences of the respective BoDV antigens of the invention. Protein modifications to the BoDV antigens of the present invention, which may occur through substitutions in amino acid sequence, and nucleic acid sequences encoding such molecules, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. In some embodiments this amendment will not significantly alter the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, Ile and Leu; the non-polar aromatic amino acids Phe, Trp and Tyr; the neutral polar amino acids Ser, Thr, Cys, Gin, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

As explained herein, in the context of the invention the BoDV antigens or proteins of the invention may be provided at the protein level or in the form of one or more nucleic acids encoding the respective BoDV protein construct, which may comprise more than one protein.

Nucleic acid sequences of the invention include the nucleic acid sequences encoding BoDV antigen, which may be processed after expression to form one or more fragments of the respective antigen or protein. Protein sequences according to *Table* 1 and functionally analogous sequences represent preferred BoDV antigens of the invention or parts thereof. Preferred nucleic acid sequence encoding BoDV antigens of the invention or parts thereof are listed under *Table 2.*

The BoDV antigens of the invention may include proteins tags that allow easy identification or binding of the provided BoDV antigens through standard techniques, for example by using antibodies directed against the protein tag. A preferred protein-tag that can be encoded by a nucleic acid sequence of the invention is a V5-tag, myc-tag, HA-tag, HIS-tag or an antibody Fc-Fragment. Alternative tags may be used instead of a V5-tag. Such alternatives are well known in the art and can be selected by a skilled person.

In another aspect, the invention encompasses BoDV antigens as disclosed herein as well as their use in the context of the methods disclosed herein. In particular, the invention also relates to nucleic acid molecules encoding BoDV antigens of the invention, and in particular one or more nucleic acid molecules encoding such BoDV antigens or parts or fragments thereof, selected from the group comprising:
a) one or more nucleic acid molecules comprising a nucleotide sequence which encodes a BoDV-GP or fragments thereof and optionally also other BoDV antigens, for example one or more epitopes of a BoDV antigen, in particular one or more epitopes of BoDV-GP or a fragment thereof;
b) one or more nucleic acid molecules which are complementary to the nucleotide sequences in accordance with a);
c) one or more nucleic acid molecules which undergo hybridization with the nucleotide sequences according to a) or b) under stringent conditions;
d) one or more nucleic acid molecules comprising a nucleotide sequence having sufficient sequence identity to be functionally analogous the nucleotide sequences according to a), b) or c);
e) one or more nucleic acid molecules which, as a consequence of the genetic code, are degenerated into nucleotide sequences according to a) through d); and
f) one or more nucleic acid molecules according the nucleotide sequences of a) through e) which are modified by deletions, additions, substitutions, translocations, inversions and/or insertions and functionally analogous to a nucleotide sequence according to a) through e)

Furhtermore, the invention encompasses nucleic acid molecules with at least 60%, preferably 70%, more preferably 80%, especially preferably 90% sequence identity to the nucleic acid molecule encoding BoDV antigens of the invention or parts thereof.

Sequence variants of the claimed nucleic acids and/or proteins, for example defined by the provided % sequence identity, that maintain the said properties of the invention are also included in the scope of the invention. Such variants, which show alternative sequences, but maintain essentially the same properties, such as autoantibody-binding properties of the respective BoDV antigen of the invention, as the specific sequences provided are known as functional analogues, or as functionally analogous. Sequence identity relates to the percentage of identical nucleotides or amino acids when carrying out a sequence alignment, for example using software such as BLAST.

It will be appreciated by those of ordinary skill in the art that, as a result of the degeneracy of the genetic code, there are many nucleotide sequences that encode a polypeptide as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions and other changes in sequence that fall under the described sequence identity are also encompassed in the invention.

Suitable solid phases for immobilizing BoDV antigens in the context of the present invention comprise, without limitation, solid beads or particles or a solid surface, such as the surface of a microtiter plate or a plate or any type of surface, which can be used in a column or a microfluidic system. A microtiter plate may be a plate made out of plastic, preferably polystyrene that has wells on its surface that can be used to run an immunoassay reaction. A solid particle or bead in the sense of the present invention can be for example a magnetic bead, a microbead, a nanobead or a nanoparticle, such as a metal nanoparticle comprising for example gold, silver, gold, ceramic or glass. Solid phases of the invention can be flat or planar or spherical or can have any kind of shape suitable for the respective application. For example, the use of a solid phase of the invention in the context of microfluidic or automated system may require a specific shape. Furthermore, the solid phase of the invention can a glass slide or a membrane, such as a PDDF or nitrocellulose membrane or other membranes commonly used in biotechnological applications.

In an advantageous embodiment of the invention, a serological assay or an immuno-assay, such as an ELISA-based method, is used for the detection of BoDV antibodies to which end binding of one or more BoDV antigens including BoDV-GP to a solid phase is performed. Following addition of sample solution, the BoDV antibodies of that sample bind to the respective antigen. The bound BoDV antibody is subsequently detected using a label, or labelled reagent and optionally quantified.

The term "in vitro method" relates to a method that is performed on a sample, for example, without limitation, a tissue or a bodily fluid, outside of the outside their normal biological context.

The term "sample" includes any biological specimen obtained from an individual. Suitable samples for use in the present invention include, without limitation, whole blood, plasma, serum, saliva, urine, stool, tears, any other bodily fluid, pure pancreatic juices or duodenal juices, tissue samples (e.g., biopsy) and cellular extracts thereof (e.g., red blood cellular extract). In a preferred embodiment, the sample is a serum sample. The use of samples such as serum, saliva, and urine is well known in the art (see, e.g., Hashida et al., J. Clin. Lab. Anal., 11:267-86 (1997)). One skilled in the art will appreciate that samples such as serum samples can be diluted prior to analysis.

As used herein, the term "antibody" includes a population of immunoglobulin molecules, which can be polyclonal or monoclonal and of any isotype, or an immunologically active fragment of an immunoglobulin molecule. Such an immunologically active fragment contains the heavy and light chain variable regions, which make up the portion of the antibody molecule that specifically binds an antigen. For example, an immunologically active fragment of an immunoglobulin molecule known in the art as Fab, Fab' or F(ab')2 is included within the meaning of the term antibody.

The term "monoclonal antibody" refers to antibodies that are made by identical immune cells that are all clones of a unique parent cell, in contrast to polyclonal antibodies, which are made from several different immune cells. Monoclonal antibodies can have monovalent affinity, in that they bind to the same epitope (the part of an antigen that is recognized by the antibody). Engineered bispecific monoclonal antibodies also exist, where each "arm" of the antibody is specific for a different epitope. Given almost any substance, it is possible to produce monoclonal antibodies that specifically bind to that substance; they can then serve to detect or purify that substance.

The term "individual," "subject," or "patient" typically refers to humans, but also to other animals including, e.g., other primates, rodents, canines, felines, equines, ovines, porcines, horeses and the like.

In a preferred embodiment, the method of the invention is for
- diagnosis of a neurological condition, encephalitis, limbic encephalitis, PNS, encephalomyelitis, leukoencephalopathy, retinitis and/or optic atrophy,
- for predicting neurological post-transplant complications or for screening organ donors or donor organs or blood donors, and/or
- for differentiating an autoimmune and an infection-related encephalitis.

The term "limbic encephalitis", as used herein, is understood to mean a neurological disease of the central nervous system, in which an MRI examination shows an abnormal signal intensity in the brain areas belonging to the limbic system, particularly temporo-medial signal rises, more particularly those which do not normalize within a few days. Even more preferably, at least one of the three symptoms from the group consisting of short-term memory disorder, temporal lobe seizures and affect disorder occurs. Characteristic MRI recordings are disclosed in the literature, for example in Simon/Greenberg/Aminoff, Clinical Neurology, 7th edition, 2009, on page 66.

The terms "diagnosis" and "diagnosing" include the use of the devices, methods, and systems, of the present invention to determine the presence or absence or likelihood of presence or absence of a medically relevant disorder in an individual, such as a BoDV infection. The term also includes devices, methods, and systems for assessing the level of disease activity in an individual. In embodiments, the term "diagnose", can be understood to mean a procedure in which information is obtained that assists the assessment, or allows the assessment in the first place, as to whether a subject is suffering from a disease or is suffering from a disease with a higher probability than an average person or suffered in the past or will suffer in the future, and also as to whether a disease is progressing or how it will develop in the future, or in order to assess whether a certain treatment is working. For example, the detection of a BoDV in a sample from a donor or a donor organ shows that the probability of post-transplantation complications caused by a BoDV is increased. Furthermore, such a detection shows that a patient can benefit from an antiviral treatment, for example with ribavirin, but not from an immunosuppressive treatment, which is aimed at preventing or reducing the formation of autoantibodies. In other words, the term "diagnose" encompasses not only making a diagnosis, but also prognostication and monitoring of the progress of a disease or of the success of therapy in the case of a disease. It can be sufficient for the diagnosis to merely detect whether the antibody is present, it being possible to determine whether detectable concentrations of the antibody are present in the sample. In embodiments, what is determined is whether the relative concentration of the antibody in a patient to be diagnosed is higher than in an average healthy person. What can be determined is whether the concentration is higher by a factor of 1.1, more preferably 1.2, 1.5, 2, 5, 10, 20, 25, 50, 100, 200, 500, 1000, 10000 or 100000, than in a sample from an average healthy person.

If an antibody against a bornavirus, can be detected, this indicates an increased probability that the patient is suffering from a BoDV infection. In embodiments, this can also indicate that an encephalitis, preferably limbic encephalitis, is caused by a viral infection, not by an autoimmune disease. Furthermore, the detection of such an antibody in a patient suffering from encephalitis can indicate that said patient is not suffering from a paraneoplastic syndrome (PNS) and thus from a PNS-associated cancer, for example a small-cell cancer of the lungs.

The detection of an antibody against a BoDV, and the infection with a BoDV to be inferred therefrom, argues for a therapeutic or precautionary treatment with an antiviral medicament or respective therapeutic actions. For example, it has been possible to show that ribavirin is effective against BoDV. On the other hand, the administration of immunosuppressants would be completely failing, since these are not only associated with significant side effects, but weaken the immune system of the patient against viral and opportunistic infections. The absence of an antibody against a BoDV argues for a therapeutic or precautionary treatment with an immunosuppressant, for example from the group comprising rituximab, prednisolone, methylprednisolone, cyclophosphamide, mycophenolate mofetil, intravenous immunoglobulins, tacrolimus, ciclosporin, methotrexate and azathioprine.

A person skilled in the art understands that such a detection generally does not allow a comprehensive diagnosis on its own, but that further aspects must be considered, for example further parameters to be determined in a sample, medical history, clinical symptoms, anamnesis or the results from imaging methods, for example MRI. Said person further understands that the value of the method according to the invention can consist in it being possible to carry out an indirect diagnosis or differential diagnosis in which the ruling out of a disease indicates that the patient is suffering from another disease with similar symptoms. In a preferred embodiment, the detection of an antibody against a BoDV can indicate that the patient is suffering from a limbic encephalitis which is not an autoimmune disease. Furthermore, such a detection can indicate that the patient is not suffering from a paraneoplastic syndrome and/or a cancer, preferably a small-cell lung cancer. Conversely, the detection of an autoantibody against the NMDA receptor can indicate that the patient is not suffering from a virally caused encephalitis, particularly limbic encephalitis. In a preferred embodiment, a person skilled in the art attaches importance to clinical symptoms mentioned herein, as is apparent from relevant textbooks on the priority date, for example Simon/Greenberg/Aminoff, Clinical Neurology, 7th edition, 2009.

The term "post-transplantation complications", as used herein, is understood to mean complications after transplantation of a body part, preferably of a tissue or solid organ, even more preferably of a solid organ which is even more preferably selected from the group comprising liver, kidney, lung, intestine, pancreas and heart. In a particularly preferred embodiment, the complications encompass the occurrence of neurological symptoms and can encompass an encephalitis, preferably limbic encephalitis. In a further particularly preferred embodiment, infections occur in the case of "post-transplantation complications". In a preferred embodiment, a neurological symptom is selected from the group comprising tetraplegia, neuropathy, clouding of consciousness, dysarthria, bradykinesia, tremor, unsteady gait, impaired cognitive skills and memory and neuritis. In a particularly preferred embodiment, the post-transplantation complications encompass an impairment of visual function, preferably neurological ophthalmological symptoms and/or diseases, particularly preferably an optic atrophy. In terms of time, "post-transplantation complications" are preferably complications which occur after the surgical procedure on the recipient, preferably up to one month, in the first six months or later than six months after the procedure.

For the screening of organ donors or donor organs, a suitable sample can be collected from the donors or their organs before the transplantation is carried out. In the case of donors, preference is given to a blood, saliva or CSF sample. In the case of organs, what are tested are blood or tissue residues, respectively samples. The tissue can originate from an organ selected from the group comprising liver, kidney, lung, intestine, pancreas, heart and brain.

"Autoimmune encephalitis", as used herein, is understood to mean an encephalitis associated with the occurrence of one or more than one autoantibody. The autoantibody is preferably an autoantibody against a neuronal autoantigen, preferably selected from the group comprising NMDAR (EP2057466 B1), GABA(A) (EP2863231 A1), GABA(B) (EP2483417 B1), DPPX (U.S. Pat. No. 9,719,993 BB), Lgl1 or CASPR2 (U.S. Pat. No. 9,250,250 BB), IGLON5 (EP2905622 A1), SNARE (EP17001205), NBC1 (EP3026434 A1), flotillin (EP3101424 A1), DAGLA (EP18196867), SNARE (EP17001205) and ITPR (EP3018478 A1). Appropriate sequences of autoantigens are disclosed in the patent specifications indicated between parentheses. Autoimmunity-caused encephalitis, more particularly NMDA receptor encephalitis, but not an encephalitis caused by infection with a BoDV, is frequently associated with the occurrence of tumors. In a preferred embodiment, the term "PNS" (paraneoplastic neurological syndrome), as used herein, is understood to mean a systemic disease which is indirectly caused by the presence of a tumor, for example by the tumor producing epitopes or releasing substances such as hormones or releasing them in an elevated quantity, which the cell from which the tumor is derived would not release or would not release in such quantities under comparable circumstances. The direct or indirect consequence may be the formation of autoantibodies which are preferentially directed against structures or parts of the nervous system, and which are in any case directly or indirectly associated with the occurrence of neurological symptoms and preferentially cause them. It is known that NMDA receptor encephalitis may be associated with the occurrence of teratomas of the ovaries. The tumor may be undetectable in the case of an autoimmunity-caused encephalitis.

The term "affinity reagent" in the context of the present invention relates to an antibody, peptide, nucleic acid, small molecule, or any other molecule that specifically binds to a target molecule in order to identify, track, capture, or influence its activity. The term "capturing" refers to binding of a target molecule by an affinity reagent.

The term "secondary affinity reagent" refers to any affinity reagent according to the above definition, which is used to bind to an antigen that is already bound by another affinity reagent.

Thus, according to the invention, detection of BoDV specific antibodies in the methods of the invention can be effected using labelled reagents according to the well-known ELISA (Enzyme-Linked Immunosorbent Assay) technology. Labels according to the invention therefore comprise enzymes catalyzing a chemical reaction which can be determined by optical means, especially by means of chromogenic substrates, chemiluminescent methods or fluorescent dyes. In another preferred embodiment BoDV specific antibodies are detected by labelling with weakly radioactive substances in radioimmunoassays (RIA) wherein the resulting radioactivity is measured.

In preferred embodiments of the invention, detection is performed by Horseradish peroxidase-(HRP)-labeled detector molecules and a HRP-catalyzed substrate reaction. At the site of reaction blue spots are developed by precipitated product from colorless substrate solution. Color intensity is correlated to the analyte concentration. Pale blue to dark blue spots are visible by eye. This technology commonly used in SeraSpot® assay and microtiter plates of the applicant are well described in the art and can be applied to various embodiments of the invention.

As examples of means for detecting antibodies bound to BoDV antigens immobilized on the solid surface of the invention for example by using a label or a signal emitted from a secondary affinity reagent, a variety of immunoassay techniques, including competitive and non-competitive immunoassays, can be used to determine the presence or level of one or more markers in a sample (see, e.g., Self et al., Curr. Opin. Biotechnol., 7:60-65 (1996)). The term immunoassay encompasses techniques including, without limitation, enzyme immunoassays (EIA) such as enzyme multiplied immunoassay technique (EMIT), enzyme-linked immunosorbent assay (ELISA), antigen capture ELISA, sandwich ELISA, IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); lateral flow tests; and chemiluminescence assays (CL).

In another preferred embodiment of the method according to the invention BoDV specific antibodies are detected in a lateral flow test, which can also be referred to as immunochromatographic test or lateral flow immunochromatographic test. Lateral flow tests are preferably based on a series of capillary beds, such as, for example, pieces of porous paper, microstructured polymer, or sintered polymer. Each of these elements has the capacity to transport fluid spontaneously. The first element (the sample pad) acts as a sponge and holds an excess of sample fluid. Once soaked, the fluid migrates to the second element (conjugate pad) in which the manufacturer has stored the so-called conjugate, a dried format of bio-active particles (see below) in a salt-sugar matrix that contains everything to guarantee an optimized chemical reaction between the target molecule (e.g., an antigen) and its chemical partner (e.g., antibody) that has been immobilized on the particle's surface. While the sample fluid dissolves the salt-sugar matrix, it also dissolves the particles and in one combined transport action the sample and conjugate mix while flowing through the porous structure. In this way, the analyte binds to the particles while migrating further through the third capillary bed. This material has one or more areas (often called stripes) where a third molecule has been immobilized by the manufacturer. By the time the sample-conjugate mix reaches these strips, analyte has been bound on the particle and the third 'capture' molecule binds the complex. After a while, when more and more fluid has passed the stripes, particles accumulate and the stripe-area changes colour. Typically there are at least two stripes: one (the control) that captures any particle and thereby shows that reaction conditions and technology worked fine, the second contains a specific capture molecule and only captures those particles onto which an analyte molecule has been immobilized. After passing these reaction zones the fluid enters the final porous material, the wick that simply acts as a waste container. Lateral Flow Tests can operate as either competitive or sandwich assays and can in principle be performed by using any coloured particle. However latex (blue colour) or nanometer sized particles of gold (red colour) are commonly used. The gold particles are red in colour due to localised surface plasmon resonance. Fluorescent or magnetic labelled particles can also be used, however these require the use of an electronic reader to assess the test result.

If desired, such immunoassays can be automated. Immunoassays can also be used in conjunction with laser induced fluorescence (see, e.g., Schmalzing et al., Electrophoresis, 18:2184-2193 (1997); Bao, J. Chromatogr. B. Biomed. Sci., 699:463-480 (1997)). Liposome immunoassays, such as flow-injection liposome immunoassays and liposome immunosensors, are also suitable for use in the present invention (see, e.g., Rongen et al., J. Immunol. Methods, 204:105-133 (1997)). In addition, nephelometry assays, in which the formation of protein/antibody complexes results in increased light scatter that is converted to a peak rate signal as a function of the marker concentration, are suitable for use in the present invention. Nephelometry assays are commercially available from Beckman Coulter (Brea, Calif.; Kit #449430) and can be performed using a Behring Nephelometer Analyzer (Fink et al., J. Clin. Chem. Clin. Biol. Chem., 27:261-276 (1989)).

In another preferred embodiment of the method according to the invention BoDV specific antibodies are detected in an immunoassay, preferably with direct or indirect coupling of one reactant to a labelling substance. This enables flexible adaptation of the method to the potentials and requirements of different laboratories and their laboratory diagnostic equipment. In one advantageous embodiment BoDV specific antibodies are detected in an immunoassay wherein the antibodies are present dissolved in a liquid phase, preferably diluted in a conventional buffer solution well-known to those skilled in the art or in an undiluted body fluid.

In another preferred embodiment of the invention, soluble or solid phase-bound antibodies are used to bind to BoDV specific antibodies. Such antibodies can be specific for IgG, IgM or IgA of the species of the subject, for example against human IgG, IgM or IgA. Furthermore, secondary affinity reagents can be employed, preferably for amplifying the signal generated by the detection reagents. Detection reagents, such as antibodies directed against BoDV specific antibodies, are detectably labelled conjugates of two components which can be conjugated with any conventional labelling enzymes, especially chromogenic and/or chemiluminescent substrates, preferably with horseradish peroxidase, alkaline phosphatase. The advantage of this embodiment lies in the use of ELISA technology usually available in laboratory facilities so that detection according to the invention can be established in a cost-effective manner. In another preferred embodiment of the invention secondary affinity reagent is detectably coupled to fluorescein isothiocyanate (FITC). Much like the above-mentioned ELISA, the FITC technology represents a system that is available in many places and therefore allows smooth and low-cost establishment of the inventive detection in laboratory routine. However, other standard fluorescence labels can also be used.

The term "serological diagnosis" or "serological test" refers to diagnostic tests or methods, which examine serum, bodily fluids or other biological samples for the presence of certain components through laboratory examination of antigen-antibody reactions in the serum. Serological techniques used for the analysis include, without limitation, ELISA, agglutination, precipitation, complement-fixation, and fluorescent antibodies.

A "kit", such as a kit for the detection of BoDV antibodies or a kit for diagnosis of BoDV infection includes all necessary analyte specific reagents required for carrying out a diagnostic test. It may also contain instructions on how to conduct the test using the provided reagents.

Specific immunological binding of an affinity reagent such as an antibody to the marker of interest can be detected directly or indirectly via a label that is emitting a signal. Any given means for detecting these labels may be considered means for detecting the label according to the method of the invention. Direct labels include fluorescent or luminescent tags, metals, dyes, radionuclides, and the like, attached to the antibody. An antibody labeled with iodine-125 (1251) can be used for determining the levels of one or more markers in a sample. A chemiluminescence assay using a chemiluminescent antibody specific for the marker is suitable for sensitive, non-radioactive detection of marker levels. An antibody labeled with fluorochrome is also suitable for determining the levels of one or more markers in a sample. Examples of fluorochromes include, without limitation, DAPI, fluorescein, Hoechst 33258, R-phycocyanin, B-phycoerythrin, R-phycoerythrin, rhodamine, Texas red, and lissamine. Secondary antibodies linked to fluorochromes can be obtained commercially, e.g., goat F(ab')2 anti-human IgG-FITC is available from Tago Immunologicals (Burlingame, Calif.).

Indirect labels include various enzymes well-known in the art, such as horseradish peroxidase (HRP), alkaline phosphatase (AP), β-galactosidase, urease, and the like. A horseradish-peroxidase detection system can be used, for example, with the chromogenic substrate tetramethylbenzidine (TMB), which yields a soluble product in the presence of hydrogen peroxide that is detectable at 450 nm. An alkaline phosphatase detection system can be used with the chromogenic substrate p-nitrophenyl phosphate, for example, which yields a soluble product readily detectable at 405 nm. Similarly, a β-galactosidase detection system can be used with the chromogenic substrate o-nitrophenyl-β-D-galactopyranoside (ONPG), which yields a soluble product detectable at 410 nm.

Methods for determining the concentration of specific molecules of interest in a sample a known to the person skilled in the art. For example, the concentration of the molecule of interest, for example a BoDV specific antibody, in a sample is determined by comparing the signal generated by a secondary affinity reagent according to the present invention that has captured the molecule of interest in said sample to the signal generated by a secondary affinity reagent that has captured the molecule of interest in a control sample, wherein the concentration of the molecule of interest in said control sample is known.

The method as described herein may also be described in terms of determining the amount of BoDV specific antibodies, such as antibodies specific for BoDV-GP, as an alternative or supplementary description to determining the concentration of BoDV specific antibodies.

"Plate readers", also known as microplate readers or microplate photometers, are instruments which are used to detect biological, chemical or physical events of samples in microtiter plates. They are widely used in research, drug discovery, bioassay validation, quality control and manufacturing processes in the pharmaceutical and biotechnological industry and academic organizations. Sample reactions can be assayed, for example, without limitation, in 6-1536 well format microtiter plates. Common detection modes for microplate assays are, for example, without limitation, absorbance, fluorescence intensity, luminescence, time-resolved fluorescence, and fluorescence polarization.

A "camera device" in the context of the present invention is a device suitable for detection of the signal for example of the labeled secondary affinity reagent directed against the antibodies bound to the immobilized BoDV antigens. The camera device can be provided as being comprised in the plate reader or individually. The person skilled in the art is familiar with such devices, which are selected based on the label of the means for detection.

A signal from the direct or indirect label can be analysed, for example, using a spectrophotometer to detect colour from a chromogenic substrate; a radiation counter to detect radiation such as a gamma counter for detection of 1251; or a fluorometer to detect fluorescence in the presence of light of a certain wavelength. For detection of enzyme-linked antibodies, a quantitative analysis of the amount of marker levels can be made using a spectrophotometer such as an EMAX Microplate Reader (Molecular Devices; Menlo Park, Calif.) in accordance with the manufacturer's instructions. If desired, the assays of the present invention can be automated or performed robotically, and the signal from multiple samples can be detected simultaneously.

In the context of the invention, the one or more BoDV antigens can be immobilized or coupled to a solid phase. Binding of the BoDV antigens disclosed herein to the solid phase can be effected via a spacer. All those chemical compounds having suitable structural and functional preconditions for spacer function can be used as spacers as long as they do not modify the binding behaviour in such a way that binding of the BoDV specific antibody to BoDV antigens disclosed herein is adversely affected.

In embodiments, the BoDV antigens can be immobilized on the solid phase through binding to organic, inorganic, synthetic and/or mixed polymers, preferably agarose, cellulose, silica gel, polyamides and/or polyvinyl alcohols.

In the meaning of the invention, immobilization is understood to involve various methods and techniques to fix the peptides on specific carriers, e.g. according to WO 99/56126 or WO 02/26292. For example, immobilization can serve to stabilize the peptides so that their activity would not be reduced or adversely modified by biological, chemical or physical exposure, especially during storage or in single-batch use. Immobilization of the peptides allows repeated use under technical or clinical routine conditions; furthermore, a sample - preferably blood components - can be reacted with at least one of the peptides according to the invention in a continuous fashion. In particular, this can be achieved by means of various immobilization techniques, with binding of the peptides to other peptides or molecules or to a carrier proceeding in such a way that the three-dimensional structure - particularly in the active center mediating the interaction with the binding partner - of the corresponding molecules, especially of said peptides, would not be changed. Advantageously, there is no loss in specificity to the BoDV antigens as a result of such immobilization. In the meaning of the invention, three basic methods can be used for immobilization:
(i) Crosslinking: in crosslinking, the peptides are fixed to one another without adversely affecting their activity. Advantageously, they are no longer soluble as a result of such crosslinking.
(ii) Binding to a carrier: binding to a carrier proceeds via adsorption, ionic binding or covalent binding, for example. Such binding may also take place inside microbial cells or liposomes or other membranous, closed or open structures. Advantageously, the peptides are not adversely affected by such fixing. For example, multiple or continuous use of carrier-bound peptides is possible with advantage in clinical diagnosis or therapy.
(iii) Inclusion: inclusion in the meaning of the invention especially proceeds in a semipermeable membrane in the form of gels, fibrils or fibers. Advantageously, encapsulated peptides are separated from the surrounding sample solution by a semipermeable membrane in such a way that interaction with the binding partner or fragments thereof still is possible. Various methods are available for immobilization, such as adsorption on an inert or electrically charged inorganic or organic carrier. For example, such carriers can be porous gels, aluminum oxide, bentonite, agarose, starch, nylon or polyacrylamide. Immobilization proceeds via physical binding forces, frequently involving hydrophobic interactions and ionic binding. Advantageously, such methods are easy to handle and have little influence on the conformation of the peptides. Advantageously, binding can be improved as a result of electrostatic binding forces between the charged groups of the peptides and the carrier, e.g. by using ion exchangers, particularly Sephadex.

Another method is covalent binding to carrier materials. In addition, the carriers may have reactive groups forming homopolar bonds with amino acid side chains. Suitable groups in peptides are carboxy, hydroxy and sulfide groups and especially the terminal amino groups of lysines. Aromatic groups offer the possibility of diazo coupling. The surface of microscopic porous glass particles can be activated by treatment with silanes and subsequently reacted with peptides. For example, hydroxy groups of natural polymers can be activated with bromocyanogen and subsequently coupled with peptides. Advantageously, a large number of peptides can undergo direct covalent binding with polyacrylamide resins. Inclusion in three-dimensional networks involves inclusion of the peptides in ionotropic gels or other structures well-known to those skilled in the art. More specifically, the pores of the matrix are such in nature that the peptides are retained, allowing interaction with the target molecules. In crosslinking, the peptides are converted into polymer aggregates by crosslinking with bifunctional agents. Such structures are gelatinous, easily deformable and, in particular, suitable for use in various reactors. By adding other inactive components such as gelatin in crosslinking, advantageous improvement of mechanical and binding properties is possible. In microencapsulation, the reaction volume of the peptides is restricted by means of membranes. For example, microencapsulation can be carried out in the form of an interfacial polymerization. Owing to the immobilization during microencapsulation, the peptides are made insoluble and thus reusable. In the meaning of the invention, immobilized peptides are all those peptides being in a condition that allows reuse thereof. Restricting the mobility and solubility of the antibodies by chemical, biological or physical means advantageously results in lower process cost.

The invention also relates to a diagnostic kit. The diagnostic kit optionally includes instructions concerning combining the contents of the kit for the detection and can also comprise instructions for diagnostic conclusions. For example, the instruction can be in the form of an instruction leaflet or other medium providing the user with information as to the type of method wherein the substances mentioned are to be used. Obviously, the information need not necessarily be in the form of an instruction leaflet, and the information may also be imparted via the Internet, for example.

The instant disclosure also includes kits, packages and multi-container units containing the herein described pharmaceutical compositions, active ingredients, and/or means for administering the same for use in the prevention and treatment of diseases and other conditions in mammalian subjects.

### FIGURES

The invention is further described by the following figures. These are not intended to limit the scope of the invention but represent preferred embodiments of aspects of the invention provided for greater illustration of the invention described herein.

### Description of the figures:

**Figure 1****:** Schematic representation of a comparative solid phase comprising BoDV proteins N, P and X. The solid phase is a so-called SeraSpot® product.
**Figure 2****:** Schematic representation of a solid phase of the invention comprising BoDV proteins N, P and GP. The solid phase is a so-called SeraSpot® product.

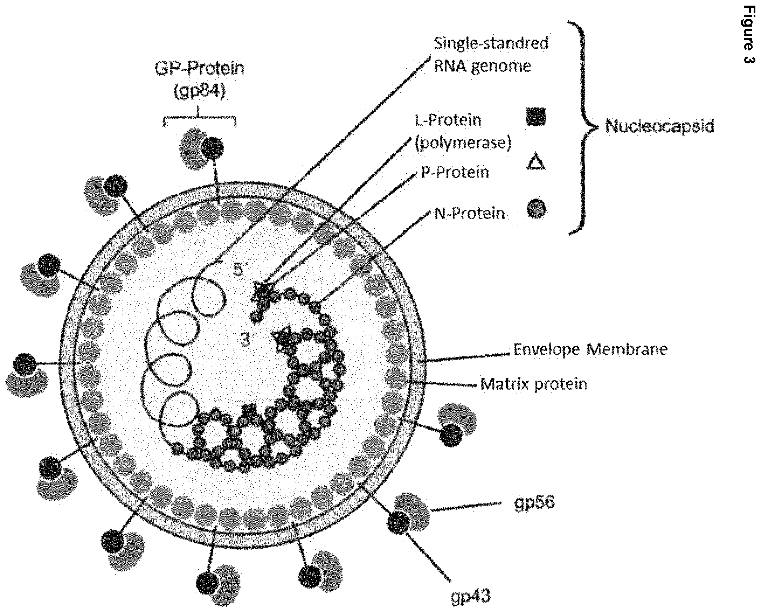

**Figure 3****:** Schematic representation of a Borna disease virus particle (taken from Modrow S. et al. (2010) Molecular Virology, 3rd edition, p.279)

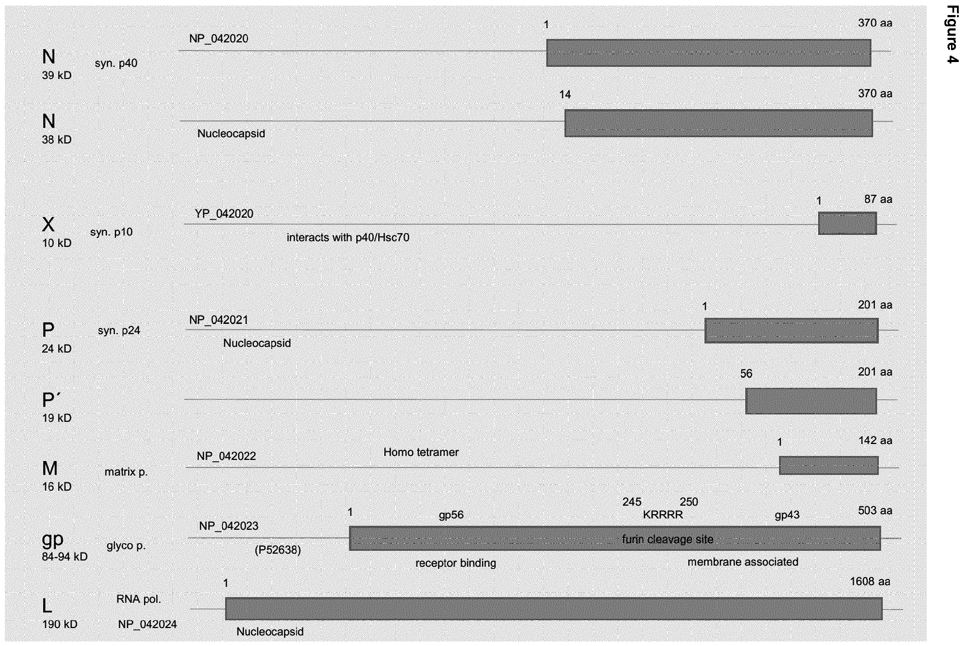

**Figure 4****:** BoDV-1 proteins and their functions deduced from the genomic organisation of Borna disease virus 1 complete sequence NC_001607.1

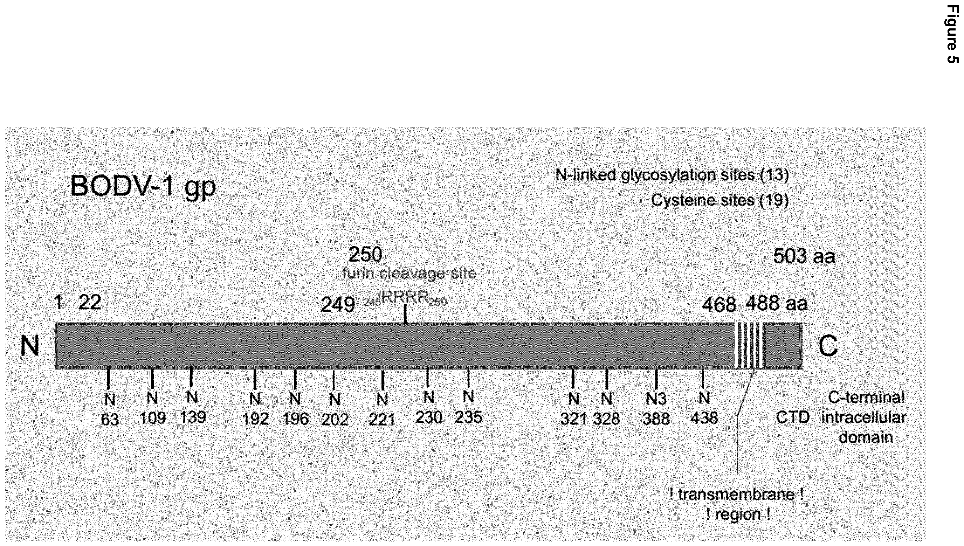

**Figure 5****:** Domain architecture and amino acid sequence features of full length BoDV-1 glycoprotein gp.

### EXAMPLES

The invention is further described by the following examples. These are not intended to limit the scope of the invention, but represent preferred embodiments of aspects of the invention provided for greater illustration of the invention described herein.

### Example 1: Expression of phosphoprotein BoDV-1 P and nucleoprotein BoDV-1 N in E.coli

The plasmids containing DNA sequences coding for phosphoprotein BoDV-1 P or nucleoprotein BoDV-1 N were generated using traditional molecular biological techniques. BoDV-1 P was cloned into the vector pGEX-4T-2 (GE Healthcare) for expression of BoDV-1 P as GST fusion. BoDV-1 N was cloned into the vector pET28a+ (Novagen) for His-tagged expression of BoDV-1 N. The first step was to generate PCR products for BoDV-1 P and BoDV-1 N with BamHI-Sall restriction ends appropriate for cloning into the respective expression vector pGEX-4T-2 (GE Healthcare) or pET28a+ (Novagen).

The sequence encoding full length BoDV-1 P (UniProt ID P0C799) was amplified by PCR using a synthetic gene for BoDV-1 P (pMAT vector; Geneart, Regensburg) as a template. The oligonucleotide primers used for amplification of BoDV-1 P were forward 5'-GAGTGGATCC **ATG** GCAACG CGA CCA TCG-3' (sense, BamHI site underlined, first codon bold; SEQ ID NO: 1), and reverse 5'-CATAGAGTCGAC **TTA** TGG TAT GAT GTC CCA TTC ATC C-3' (antisense, Sall site underlined, stop codon bold ; SEQ ID NO: 2).

The sequence encoding full length BoDV-1 N (UniProt ID P0C797) was amplified by PCR using a synthetic gene for BoDV-1 N (pMAT vector; Geneart, Regensburg) as a template. The oligonucleotide primers used for amplification of BoDV-1 N were forward 5'-GAGTGGATCC **ATG** CCA CCC AAG AGA CGC-3' (sense, BamHI site underlined, first codon bold; SEQ ID NO: 3), and reverse 5'-CATAGAGTCGAC **TTA** GTT TAG ACC AGT CAC ACC TAT CA-3' (antisense, Sall site underlined, stop codon bold; SEQ ID NO: 4).

The PCR amplification reaction for BoDV-1 P and BoDV-1 N each contained 0.1 ng pMAT vector (Geneart, Regensburg) including the respective synthetic gene as template, 50 pmoles of each primer forward and reverse, I0 µl of 10X Buffer with MgCl₂, 16 µl of dNTPs 1.25mM each, H₂O up to I00 µl and 0.75 µL DNA polymerase from Roche Expand High Fidelity PCR kit. Amplification conditions: 5' 95°C, 30 cycles: 30" 95°C, 1' 60°C, 2' 72°C, followed by a single step 72°C extension cycle and a 4°C hold.

The PCR reactions were purified using the Qiagen PCR Purification kit and digested with BamHI and Sall for 3 hours at 37°C with 50 u of each enzyme in a 100 µl final volume. The BamHI-Sall digested PCR products were then purified from 1% agarose GTG (genetic technology grade) using the Qiagen MinElute Gel Extraction kit.

15 ng of the BamHI-Sall gel-purified PCR product of BoDV-1 P was ligated to a dephosphorylated, gel-purified pGEX-4T-2 BamHI-Sall vector using the New England Biolabs T4 DNA Ligase kit. After 20' at RT the ligation reaction was used to transform electrocompetent *E.coli* XL1 blue cells, then plated on LB agar plates containing I00µg/ml ampicillin.

15 ng of the BamHI-Sall gel-purified PCR product of BoDV-1 N was ligated to a dephosphorylated, gel-purified pET28a+ BamHI-Sall vector using the New England Biolabs T4 DNA Ligase kit. After 20' at RT the ligation reaction was used to transform electrocompetent *E.coli* XL1 blue cells, then plated on LB agar plates containing 30µg/ml kanamycin.

Miniprep plasmid DNA was prepared from single antibiotic resistant colonies grown in Luria Broth containing the corresponding antibiotic; a portion of the plasmid DNA was digested with BamHI+Sall to confirm the presence of the inserts. The plasmid from a positive clone was analysed by DNA sequencing.

Next, the sequence verified plasmids from *E.coli* XL1 blue were subcloned into *E.coli* strains suitable for recombinant protein production. A sequence verified pGEX-4T-2 plasmid containing full length BoDV-1 P was used to transform electrocompetent *E.coli* BL21 cells. A sequence verified pET28a+ plasmid containing full length BoDV-1 N was used to transform electrocompetent *E.coli* BL21DE3 cells. After electroporation cells were plated on LB agar plates containing 100µg/ml ampicllin for selection of pGEX-4T-2 containing clones or 30µg/ml kanamycin for selection of pET28a+ containing clones. The plasmid from a single growing clone was analysed by DNA sequencing again.

For production of recombinant BoDV-1 protein in *E.coli,* a preculture was inoculated from a glycerol stock of a single clone and grown in Terrific broth at 37°C overnight. The preculture was diluted 1:50 in 1 I Erlenmeyer flasks containing Terrific broth with a blend of glucose and lactose as carbon source for autoinduction. The culture was shaken at 25°C for 24 hours at 220 rpm in orbital shakers. Induction occurred upon depletion of the glucose from the media. Cells were then harvested by low-speed centrifugation at 3500xg (Sorvall Lynx 6000, Thermo Fisher Scientific, Germany; fixed angle rotor 6x 1000 ml) for 20 min at 4°C. Obtained cell pellets containing the expressed recombinant BoDV-1 protein were stored frozen at -70°C.

### Example 2: Expression of glycoprotein BoDV-1 GP in L.tarentolae

The expression plasmid for constitutive, secretoric expression of recombinant glycoprotein BoDV-1 GP in parasite cells Leishmania tarentolae was constructed using a commercial vector pLEXSY-sat2 (Jena Bioscience, Jena, Germany).

*L. tarentolae* cells, empty expression vector (pLEXSY-sat2) and all culture materials were purchased from Jena Bioscience (Jena, Germany). Cells were grown at 26°C in static suspension cultures in LEXSY BHI liquid medium supplemented with porcine hemin (5 µg/ml), penicillin/ streptomycin (Pen-Strep, 100 U/ml) and nourseothricin (NTC, 100 µg/ml).

The sequence encoding BoDV-1 GP aa 23-446 (UniProt ID P52638) was amplified by PCR using a synthetic gene codon-optimized for *L.tarentolae* (pMAT vector; Geneart, Regensburg) as a template. The oligonucleotide primers used for amplification of BoDV-1 GP were forward 5'-GCGTCTAGAC **TTC** GAT CTG CAG GGC CTG-3' (sense, Xbal site underlined, first codon bold; SEQ ID NO: 5), and reverse 5'-CTAGGTACC GCT ACC AGT GTC TGT GCT CG-3' (antisense, Kpnl site underlined, no stop codon; SEQ ID NO: 6).

The PCR product was digested with Xbal and Kpnl and cloned into the corresponding restriction sites of vector pLEXSY-sat2.

*L. tarentolae* cells were transfected by electroporation. pLEXSY-sat2 containing the DNA sequence of BoDV-1 GP was first digested with Swal restriction enzyme and the linearized expression cassette (without *E*. coli-relevant ori and antibiotic selection marker) was purified by Qiagen Gel Extraction kit. Parasite cells in logarithmic growth phase were centrifuged (2500xg, 5 min), resuspended in LEXSY BHI liquid medium at a final concentration of 1 × 10⁸ cells/ml and kept on ice for 10 min prior to the addition of 2.5 µg of linearized expression cassette.

Following electroporation (Eppendorf Multiporator, 2 mm cuvette, 450 V, 5 msec) and 10 min incubation on ice, cells were resuspended in fresh BHI culture medium and grown in suspension for the next 24 h. Single colony derived, NTC resistant strains were selected after 9 days of growth on solid media (BHI-agar containing 100 µg/ml NTC) and maintained in suspension culture with constant antibiotic concentration.

Genomic integration of the expression cassette BoDV-1 GP in transfected *L. tarentolae* strains was verified by diagnostic PCR reaction with primers used for amplification of BoDV-1 GP.

For production of recombinant BoDV-1 gp, parasite cells were grown at 26°C in 1 I Erlenmeyer flasks on orbital shakers (120 rpm). After 72 h of culture growth, cells were separated by low-speed centrifugation at 3500xg (Sorvall Lynx 6000, Thermo Fisher Scientific, Germany; fixed angle rotor 6x 1000 ml) for 20 min at 4°C.

Clarified cell culture supernatants were concentrated by tangential flow filtration using Vivaflow 200 modules (Sartorius, Göttingen, Germany) and concentrated supernatants containing the secreted recombinant BoDV-1 GP were immediately processed further.

### Example 3: Purification of phosphoprotein BoDV-1 P

The cell pellet was thawed and washed in cold GST lysis buffer (50 mM Tris-HCI, pH 8.0, 150 mM NaCl). Each pellet was then resuspended in a volume of cold GST lysis buffer equal to the packed cell volume, in order to normalize for differences in the density of different expression cultures. The cell/buffer suspension was lysed enzymatically assisted by ultrasound.

The crude lysate was centrifuged at 6000×g (Sorvall Lynx 6000, Thermo Fisher Scientific, Germany; fixed angle rotor 6× 1000 ml) for 30 min at 4°C to pellet the insoluble fraction.

Recombinant BoDV-1 P was purified from cleared lysate by glutathione affinity chromatography. The supernatant was incubated with 3 ml of pre-equilibrated glutathione agarose (Sigma Aldrich, Taufkirchen, Germany) on a rotating wheel for 3 h at 4°C.

The glutathione agarose with bound protein was packed in a 10 ml chromatography column (Tricorn 10/100; GE Healthcare, Freiburg, Germany). Weakly bound and contaminating proteins were washed from the agarose gel by using 10× the column volume of GST lysis buffer (50mM Tris-HCI, pH 8.0, 150 mM NaCl). The recombinant GST protein was finally eluted from the packed bed with 3x the column volume of GST elution buffer (50mM Tris-HCI, pH 9.0, 150 mM NaCl) containing 10 mM reduced glutathione.

One ml fractions were collected and the protein contents analyzed by SDS-PAGE (Serva Electrophoresis, Heidelberg, Germany). Protein containing fractions were pooled and a BCA Protein Assay (Pierce) using bovine serum albumin (BSA) as a standard reference for protein concentration determination was done.

### Example 4: Purification of nucleoprotein BoDV-1 N

The cell pellet was thawed and washed in cold NiNTA lysis buffer (20 mM Tris-HCI, pH 7.4, 300 mM NaCl). Each pellet was then resuspended in a volume of cold NiNTA lysis buffer equal to the packed cell volume, in order to normalize for differences in the density of different expression cultures. The cell/buffer suspension was lysed enzymatically assisted by ultrasound.

The crude lysate was centrifuged at 6000xg (Sorvall Lynx 6000, Thermo Fisher Scientific, Germany; fixed angle rotor 6× 1000 ml) for 30 min at 4°C to pellet the insoluble fraction.

Recombinant BoDV-1 N was purified from cleared lysate by NiNTA affinity chromatography. The supernatant was incubated with 3 ml of pre-equilibrated NiNTA agarose (Qiagen, Hilden, Germany) on a rotating wheel for 16 h at 4°C.

The NiNTA agarose with bound protein was packed in a 10 ml chromatography column (Tricorn 10/100; GE Healthcare, Freiburg, Germany). Weakly bound and contaminating proteins were washed from the agarose gel by using 10× the column volume of NiNTA lysis buffer (20mM Tris, pH 7.4, 0.3M NaCl). The recombinant His-tagged protein was finally eluted from the packed bed with 3× the column volume of NiNTA elution buffer (20mM Tris, pH 8.0, 0.3M NaCl) containing 250 mM imidazole.

One ml fractions were collected and the protein contents analyzed by SDS-PAGE (Serva Electrophoresis, Heidelberg, Germany). Protein containing fractions were pooled and a BCA Protein Assay (Pierce) using bovine serum albumin (BSA) as a standard reference for protein concentration determination was done.

### Example 5: Purification of glycoprotein BoDV-1 GP

Recombinant BoDV-1 GP from concentrated *L.tarentolae* cell culture supernatants was purified by NiNTA affinity chromatography. The supernatant was incubated with 3 ml of pre-equilibrated NiNTA agarose (Qiagen, Hilden, Germany) on a rotating wheel for 16 h at 4°C.

The NiNTA agarose with bound protein was packed in a 10 ml chromatography column (Tricorn 10/100; GE Healthcare, Freiburg, Germany). Weakly bound and contaminating proteins were washed from the agarose gel by using 10× the column volume NiNTA buffer (20mM Tris, pH 7.4, 0.3M NaCl). The recombinant His-tagged protein was finally eluted from the packed bed with 3× the column volume of NiNTA elution buffer (20mM Tris, pH 8.0, 0.3MNaCl, 250mM imidazole).

One ml fractions were collected and the protein contents analyzed using a BioDrop Touch Duo spectrophotometer (BioDrop, Serva Electrophoresis, Heidelberg, Germany). Protein containing fractions were pooled and a BCA Protein Assay (Pierce) using bovine serum albumin (BSA) as a standard reference for protein concentration determination was done. In some cases UV absorbance at 280 nm was used, assuming 1 absorbance unit = 1 mg/mL as indicated by calibration with BCA results.

**Table 3: List of oligonucleotides used for expression cloning of BoDV-1 proteins.**

| Name | Oligonucleotide sequence | SEQ ID |
|---|---|---|
| BoDV_P_aa001_Fwd | 5'-GAGTGGATCCATGGCAACGCGACCATCG-3' | NO: 1 |
| BoDV_P_aa201_Rev | 5'-CATAGAGTCGACTTATGGTATGATGTCCCATTCATCC-3' | NO: 2 |
| BoDV_N_aa001_Fwd | 5'-GAGTGGATCCATGCCACCCAAGAGACGC-3' | NO: 3 |
| BoDV_N_aa370_Rev | 5'-CATAGAGTCGACTTAGTTTAGACCAGTCACACCTATCA-3' | NO: 4 |
| BoDV_gp_aa023_Fwd | 5'-GCGTCTAGACTTCGATCTGCAGGGCCTG-3' | NO: 5 |
| BoDV_gp_aa446_Rev | 5'-CTAGGTACCGCTACCAGTGTCTGTGCTCG-3' | NO: 6 |

### Comparative example

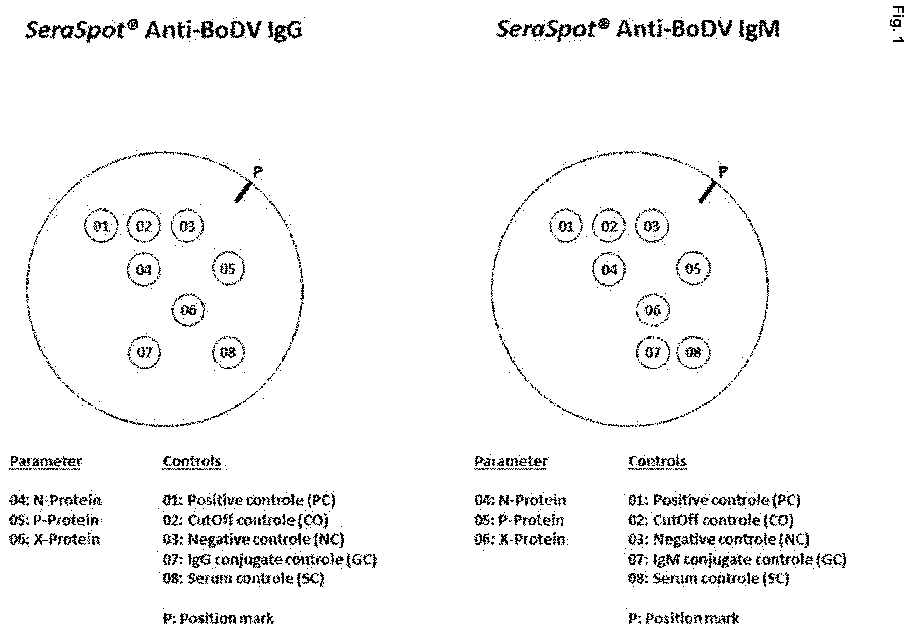

Testing of sample of BoDV infected patients using a solid phase according to Figure 1, representing a comparative example comprising BoDV protein P, N and X.

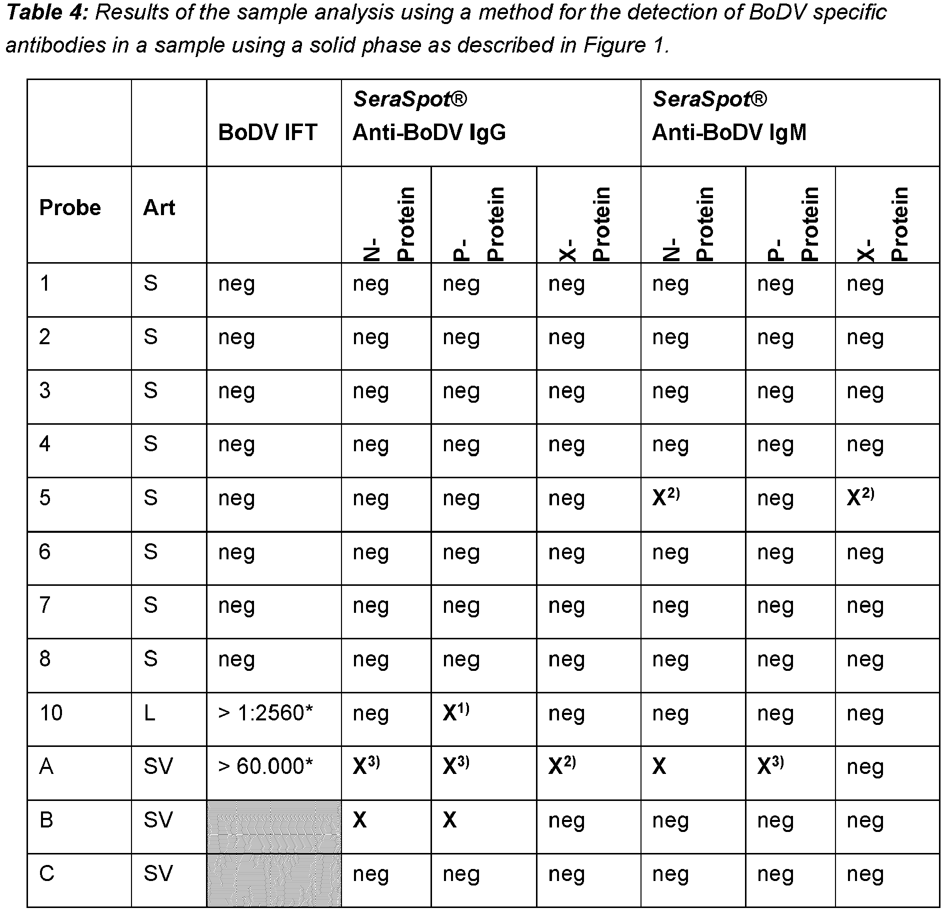

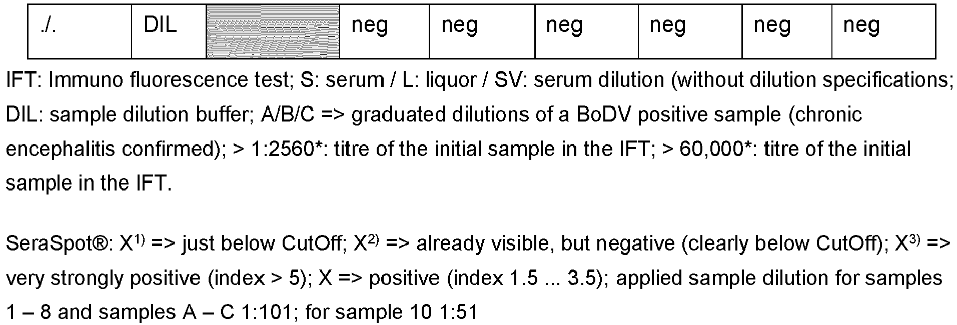

## Claims

1. A solid phase comprising one or more immobilized Borna disease virus (BoDV) antigens for the detection of BoDV specific antibodies in a sample, wherein
said one or more immobilized BoDV antigens comprise BoDV-glycoprotein (BoDV-GP) and/or fragments thereof.

2. Solid phase according to the preceding claim, comprising additionally at least one antigen selected from the group comprising BoDV-N-protein, BoDV-P-protein and BoDV-X-protein, and/or fragments thereof.

3. Solid phase according to the preceding claim, wherein each antigen is located on spatially separated area of the solid surface enabling individual and/or independent detection of antibodies directed against each antigen.

4. Solid phase according to any of the preceding claims, wherein the solid phase is flat or spherical, such as a microtiter plate, one or multiple beads, a glass slide or a membrane, and/or comprises or consists of plastic, glas, nitrocellulose, PVDF, polystyrene or latex.

5. Solid phase according to any of the preceding claims, wherein the BoDV is selected from the group comprising BoDV-1, BoDV-2 and VSBV-1.

6. Solid surface according to any of the preceding claims, wherein the BoDV-GP or fragments thereof is glycosylated.

7. Solid surface according to any of the preceding claims, wherein the BoDV-GP or fragments thereof is present as a dimer consisting of BoDV-GP fragments gp43 and gp56 or fragments thereof.

8. Solid surface according to the preceding claim, wherein gp43 and gp56 or fragments thereof are linked by at least one disulfide bond.

9. Solid surface according to any of the preceding claims, wherein the BoDV-GP is isolated from a eukaryotic protein expression system, preferably a *Leishmania tarentolae* expression system.

10. Solid surface according to any of the preceding claims, wherein the BoDV-GP and at least one of BoDV-N-protein, BoDV-P-protein and BoDV-X-protein are immobilized on spatially separated areas of a surface of a well of a microtiter plate, and wherein said surface comprises additional spatially separated areas serving as controls, for example for binding of labeled secondary affinity reagents and functionality of means for detecting a signal emitted from a labeled secondary affinity reagent.

11. In vitro method for the detection of a BoDV infection in a subject, comprising
- providing a sample of said subject,
- providing a solid phase comprising one or more immobilized BoDV antigens according to any of claims 1-10,
- contacting said sample with said solid phase, and
- detecting antibodies from said sample that bind to said one or more BoDV antigens.

12. Method according to any of the preceding claims, wherein the subject is human

13. Method according to claims 11-12,
- for the diagnosis of a neurological condition, encephalitis, limbic encephalitis, PNS, encephalomyelitis, leukoencephalopathy, retinitis and/or optic atrophy,
- for predicting neurological post-transplant complications or for screening organ donors or donor organs or blood donors, and/or
- for differentiating an autoimmune and an infection-related encephalitis.

14. An isolated BoDV-GP or fragments thereof, wherein the BoDV-GP has been purified from a *Leishmania tarentolae* expression system and is a dimer consisting of BoDV-GP fragments gp43 and gp56 or fragments thereof, wherein gp43 and gp56 or fragments thereof are linked by at least one disulfide bond.

15. A kit for the detection of BoDV specific antibodies in a sample, comprising
- a solid phase according to any of claims 1-10, or
- one or more isolated BoDV antigens comprising BoDV-GP or fragments thereof and preferably at least one or BoDV-N-protein, BoDV-P-protein and BoDV-X-protein, or fragments thereof, and a solid phase for immobilization of said one or more isolated BoDV antigens, AND
- means for detecting antibodies bound to BoDV antigens immobilized on the solid surface, such as labeled secondary affinity reagents directed against the bound antibodies and means for detecting a signal emitted from said label, AND OPTIONALLY,
- computer software configured for determining the presence and/or the amount of bound antibodies.
